# EUROPEAN PATENT SPECIFICATION

(11) **EP 3 555 319 B1**
(45) Date of publication and mention of the grant of the patent: **14.06.2023**
(21) Application number: 17829996.2
(22) Date of filing: 14.12.2017
(51) Int. Cl.: C12Q 1/6883

(54) **DIAGNOSIS OF PARKINSON'S DISEASE ON THE BASIS OF DECREASED OVERALL TRANSLATION**
DIAGNOSE DER PARKINSON-KRANKHEIT AUFGRUND EINER VERMINDERTEN GESAMTTRANSLATION
DIAGNOSTIC DE LA MALADIE DE PARKINSON SUR LA BASE D'UNE TRADUCTION GLOBALE RÉDUITE

(30) Priority: 14.12.2016 FI 20165967
(43) Date of publication of application: 23.10.2019
(73) Proprietor: Åbo Akademi University, 20500 Turku (FI)
(72) Inventor: FLINKMAN, Dani, 20500 Turku (FI); HONG, Ye, 20500 Turku (FI); DESHPANDE, Prasannakumar Shriprakash, 20500 Turku (FI); LAURÉN, Tomas Lassi-Pekka, 20540 Turku (FI); PELTONEN, Sirkku, 20500 Turku (FI); KAASINEN, Valtteri, 20500 Turku (FI); JAMES, Peter Hywel, 20500 Turku (FI); COFFEY, Eleanor Therese, 20500 Turku (FI)
(74) Representative: Kolster Oy Ab
(86) International application number: PCT/FI2017/050892
(87) International publication number: WO 2018/109277

(56) References cited:
- WO-A1-2013/098786
- IAN MARTIN: "Decoding Parkinson's Disease Pathogenesis: The Role of Deregulated mRNA Translation", JOURNAL OF PARKINSON'S DISEASE, vol. 6, no. 1, 30 March 2016 (2016-03-30), pages 17-27, XP055452323, NL ISSN: 1877-7171, DOI: 10.3233/JPD-150738
- TAYMANS JEAN-MARC ET AL: "Deregulation of protein translation control, a potential game-changing hypothesis for Parkinson's disease pathogenesis", TRENDS IN MOLECULAR MEDICINE, vol. 21, no. 8, 1 August 2015 (2015-08-01) , pages 466-472, XP009503616,
- SRIVASTAVA GARIMA ET AL: "Proteomics in Parkinson's disease: current trends, translational snags and future possibilities", EXPERT REVIEW OF PROTEOMICS, FUTURE DRUGS LTD., LONDON, GB, vol. 7, no. 1, 31 January 2010 (2010-01-31), pages 127-139, XP009503574, ISSN: 1478-9450, DOI: 10.1586/EPR.09.91
- ZHANG TERRY ET AL: "11.24 Determination of biomarker proteins for Parkinson's disease using differential quantitative proteomic analysis with clCAT and two-dimensional chromatography on a nanoelectrospray linear-lon trap LC/MS system", MOLECULAR & CELLULAR PROTEOMICS; 3RD ANNUAL WORLD CONGRESS OF THE HUMAN-PROTEOME-ORGANISATION (HUPO), AMERICAN SOCIETY FOR BIOCHEMISTRY AND MOLECULAR BIOLOGY, US; BEIJING, PEOPLES R CHINA, [Online] vol. 3, no. 10, Suppl. S, 1 October 2004 (2004-10-01), XP009503575, ISSN: 1535-9476 Retrieved from the Internet: URL:http://www.mcponline.org/content/3/10_ suppl/S135.full.pdf+html>

## Description

### FIELD OF THE INVENTION

The present invention relates to diagnosis of Parkinson's disease (PD), preferably at the pre-symptomatic stage. The invention can be utilized in methods for diagnosing PD, monitoring progression of PD, monitoring response to treatment, or stratifying subjects for clinical studies, for instance.

### BACKGROUND OF THE INVENTION

Parkinson's disease (PD) is the second most common neurodegenerative disorder after Alzheimer's disease with the highest prevalence in Europe and North and South America. No cure exists for PD, though several promising interventions are in clinical trial and long term symptomatic treatment is associated with complications. Although its neuropathological basis is not fully understood, degeneration of dopaminergic neurons in the *substantia nigra pars compacta* (SNpc) has been repeatedly shown, and the subsequent reduction of dopamine release in the basal ganglia leads to Parkinsonian motor symptoms.

PD is a heterogenous disorder that can be roughly classified as sporadic, non-inherited (95% of cases) and the inherited (familial) PD, for which 28 risk associated variants are known ¹. Key features of pathology include motor symptoms, dopaminergic neuron death, neuritic atrophy, dopamine depletion and behavioral disturbances. Interestingly, LRRK2-G2019S carriers are symptomatically indistinguishable from sporadic cases ², suggesting that there may be a common underlying mechanism. Study of LRRK2-G2019S is therefore hoped to provide insight that is relevant also to the larger population of sporadic cases.

By the time PD symptoms emerge, up to 80% of the brain's dopaminergic neurons are already dead. Replacing or regenerating these dead neurons is not feasible but the long pre-clinical phase provides the possibility for therapeutic intervention prior to extensive neuronal loss. While therapeutic strategies have been proposed based on new mechanistic insight ^{3, 4}, there are no accepted definitive biomarkers of PD. Therefore, there is an unmet need to identify biomarkers for diagnosing PD at the pre-symptomatic stage so that when a cure or preventive treatment arises it could have maximal benefit.

### BRIEF DESCRIPTION OF THE INVENTION

An object of the invention is to provide means and methods for diagnostics of PD. This object is achieved by a method and the use of a kit characterized by what is stated in the independent claims. Some preferred embodiments of the invention are disclosed in the dependent claims.

The invention allows for the detection of the disease in various tissue types in a minimally invasive fashion (i.e. using fibroblasts from a skin biopsy or the biopsy itself or from peripheral mononuclear blood cells from a blood sample or from other bodily fluids e.g. urine or cerebral spinal fluid). The readout can be in the form of a change in protein synthesis levels or by the change in expression of unique protein or peptide signatures unique to the disease. The readout is relevant for diagnosis of sporadic, non-inherited cases as well as for inherited cases, as demonstrated by examples.

In one aspect, the present invention provides a method of diagnosing or monitoring Parkinson's disease (PD) in a subject, said method comprising testing a sample obtained from the subject for decreased overall translation. Decreased overall translation is indicative of PD; whereas an absence of altered translation is indicative of the absence of PD.

Decreased overall translation may manifest itself in different ways. In some embodiments, the presence of decreased overall translation is indicated by altered level of translation as compared with that of a control sample. The overall decreased level of translation may result from a combination of increases and decreases in protein expression level for different proteins, such that the overall expression level is decreased. The level of translation may be assessed by a method selected from the group consisting of metabolic labelling, and click chemistry, for example.

In some other embodiments, the presence of decreased overall translation is indicated by the presence of a unique molecular signature. The method may or may not involve comparing the signature with that of a relevant control.

In some further embodiments, the presence of decreased overall translation is indicated by changes in post-translational modifications, which may be assessed either quantitatively or qualitatively. In some even further embodiments, changes in structure of post-translational modifications are indicative of decreased overall translation.

The method may be used for monitoring PD for different purposes. For example, the method may be used for monitoring progression of PD, monitoring response to treatment, monitoring remission of PD, or monitoring relapse of PD.

In another aspect, the present invention provides a use of a kit for diagnosing, staging or monitoring PD, or stratifying PD patients based on changes in SNX2, FAM120A or ADAR expression, wherein said kit comprises at least one or more reagents for testing a sample for changes in SNX2, FAM120A or ADAR expression.

Other objectives, aspects, embodiments, details and advantages of the present invention will become apparent from the following figures, detailed description, and examples.

### BRIEF DESCRIPTION OF THE DRAWINGS

In the following the invention will be described in greater detail by means of preferred embodiments with reference to the attached figures, in which the basis for the invention and its application in diagnosis in two formats are shown.

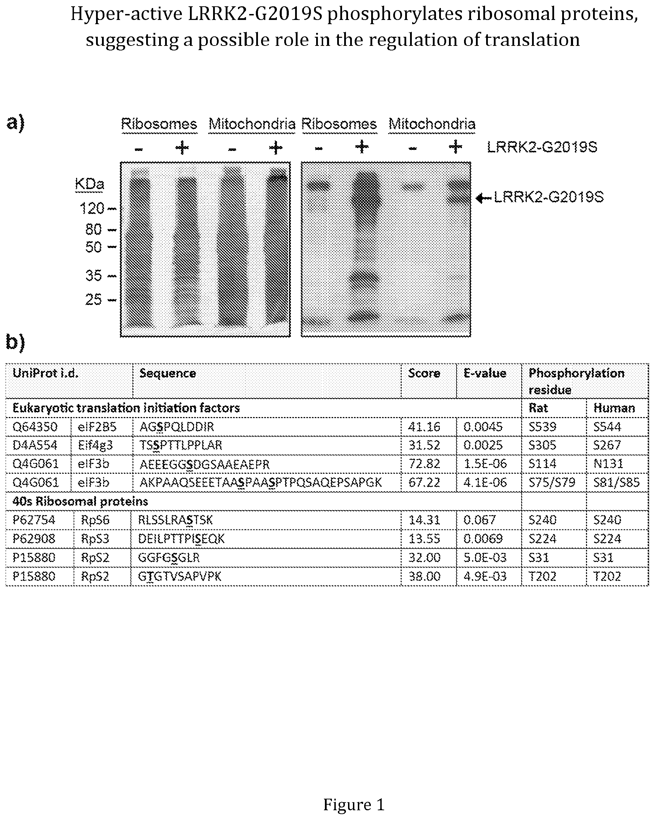

**Figure 1** shows that LRRK2-G2019S (a gain of function kinase mutant of LRRK2 (PARK8) that is associated with familial and sporadic PD) phosphorylates ribosomal proteins whereas phosphorylation of mitochondrial proteins is relatively low. a) Silver-staining (l.h.s.) of an SDS-PAGE gel shows protein loading from ribosomal and mitochondrial fractions. Autoradiographs (r.h.s.) show unexpectedly that LRRK2-G2019S preferentially phosphorylates ribosomal proteins. b) Mass spectrometric identification of LRRK2-G2019S substrates from ribosomal fractions. The precise LRRK2-G2019S phosphorylation sites are shown. These data indicate that LRRK2-G2019S regulates ribosomal function.

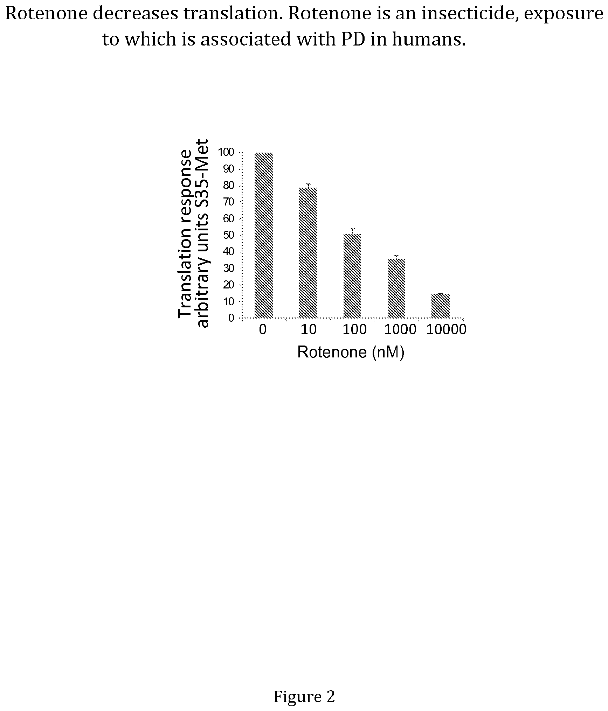

**Figure 2** demonstrates using S35-methioine labelling that protein translation is impaired in the rotenone model of Parkinson's disease (PD). Hippocampal neurons were treated with or without rotenone as indicated. Rotenone is an insecticide that upon chronic administration reproduces the hallmarks of sporadic PD in rodents. In humans, exposure to rotenone is associated with increased risk to develop PD. These data show that protein synthesis inhibition occurs in PD and could contribute to the pathology. Standard errors of the mean are shown.

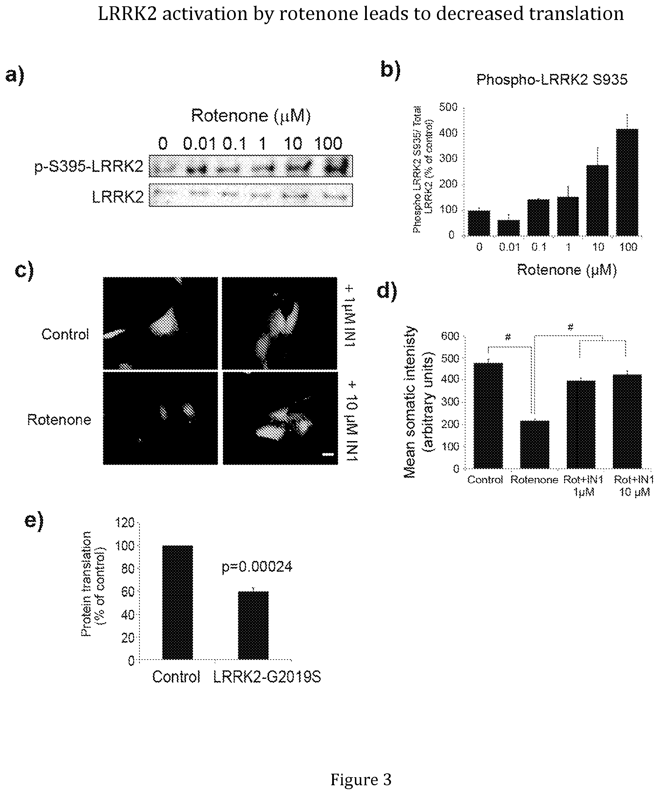

**Figure 3** demonstrates that rotenone activates endogenous LRRK2 in dopaminergic neurons and in BHK21 cells and that this can be recovered upon inhibition of endogenous LRRK2 kinase activity using LRRK2-IN1. Furthermore, LRRK2-G2019S directly impairs protein translation in a reconstituted translation system. a) LRRK2 activity is inferred using a phospho-specific antibody (LRRK2-S395) that recognises the active form of LRRK2. b) Quantitative data from 6 independent repeats as shown in (a). c, d) To determine whether inhibition of LRRK2 kinase activity could recover normal protein translation in cultured cells, BHK21 cells were treated with or without rotenone (100 nM) in the presence or absence of the indicated concentration of LRRK2-IN1. Protein translation was measured using non-canonical amino acid labelling with AHA followed by copper-catalysed click addition of alkyne-Alexa-488. Representative fluorescent micrographs (c) and quantitative data (d) are shown. Protein translation level is recovered to control level upon inhibition of LRRK2 using 1µM LRRK2-IN1. e) To directly test the effect of LRRK2-G2019S on protein synthesis, purified LRRK2-G2019S was added to a cell-free translation system and protein translation determined by colorimetric production of beta-galactosidase. These data show that LRRK2-G2019S impairs protein synthesis machinery directly, and that endogenous LRRK2 activity represses protein synthesis in response to rotenone treatment. Standard errors of the mean are plotted. #, p<0.0001.

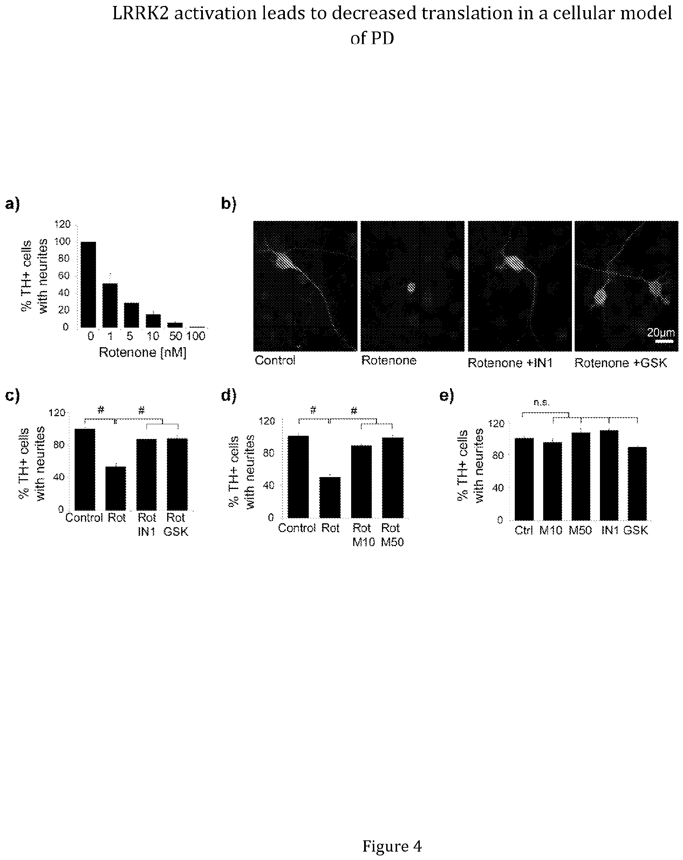

**Figure 4** shows treatment with LRRK2 kinase inhibitors protects cultured mid brain dopaminergic neurons from rotenone induced neurite atrophy. a) Midbrain cultures were treated with rotenone (0-100 nM) for 24 h as indicated. The cultures were immunostained for Tyrosine hydroxylase to identify dopaminergic neurons. The percentage of dopaminergic, TH-positive cells that had intact neurites are shown. Rotenone induced a dose dependent loss of neurites. Mean data +/- standard deviation from two independent experiments are shown. Over 300 cells were scored per condition. b) A representative fluorescence micrograph showing TH immunostaining (green) dopaminergic neurons is shown. (c,d) Treatment with three structurally different LRRK2 inhibitors, LRRK2-IN1 (1 µM), GSK-2578215A (1 µM) or MLi-2 (10 or 50 nM) prevented rotenone induced loss of neurites in the dopaminergic neurons. (e) Treatment with the inhibitors alone did not change the number of dopaminergic neurons with intact neurites. Standard errors of the mean are shown. #, p< 0.0001.

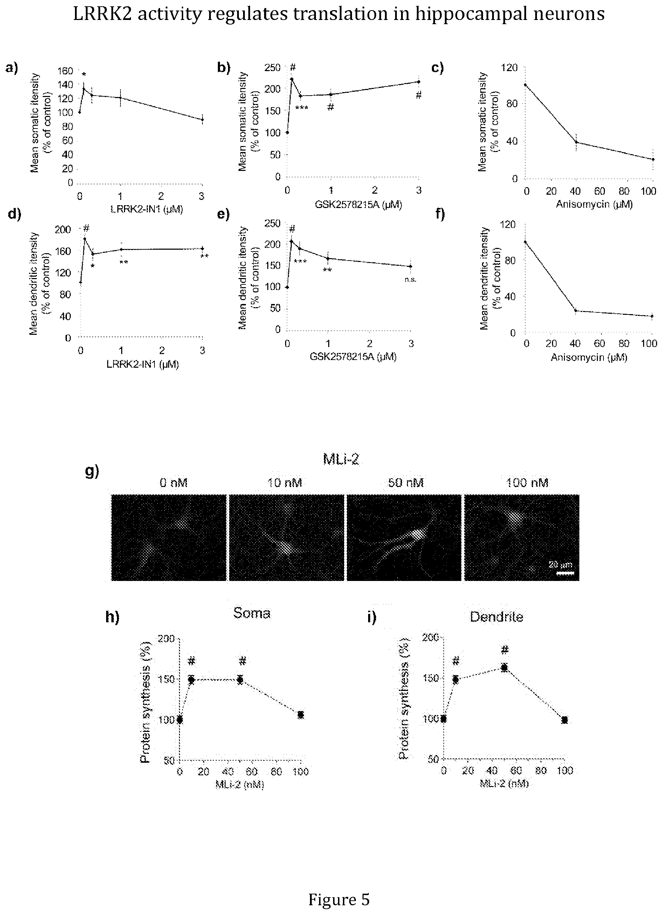

**Figure 5** shows that by using click chemistry and Alexa-fluor-488-alkyne as a reporter, that endogenous LRRK2 represses protein translation in primary cultured neurons. a-f) Hippocampal neurons were treated with or without LRRK2 inhibitors (LRRK2-IN1 or GSK-2578215A) as shown. The level of protein translation was measured following metabolic labelling of neurons with the methionine analogue L-azido-homoalanine (AHA). A click reaction was used to covalently tag nascent proteins with Alexa-fluor-488. The level of *de novo* protein synthesis was inferred from the fluorescence intensity. Treatment with two structurally independent LRRK2 inhibitors increased the level of protein synthesis in hippocampal neurons (a-f). Anisomycin, an inhibitor of protein synthesis, was added as a negative control. Standard errors of the mean are shown. *, p< 0.05; **, p< 0.01; ***, p< 0.001. In a similar way, click chemistry was used to test an orally available specific inhibitor of Lrrk2 (MLi-2). g-i) shows that treatment of cultured hippocampal neurons with highly specific inhibitor of LRRK2, MLi-2 increased *de novo* protein synthesis. g) Representative fluorescence images of hippocampal neurons treated with indicated amounts of MLi-2 are shown. (h, i) The level of *de novo* protein synthesis in soma and dendrites was inferred from the fluorescence intensity. Standard errors of the mean are shown. #, p< 0.0001.

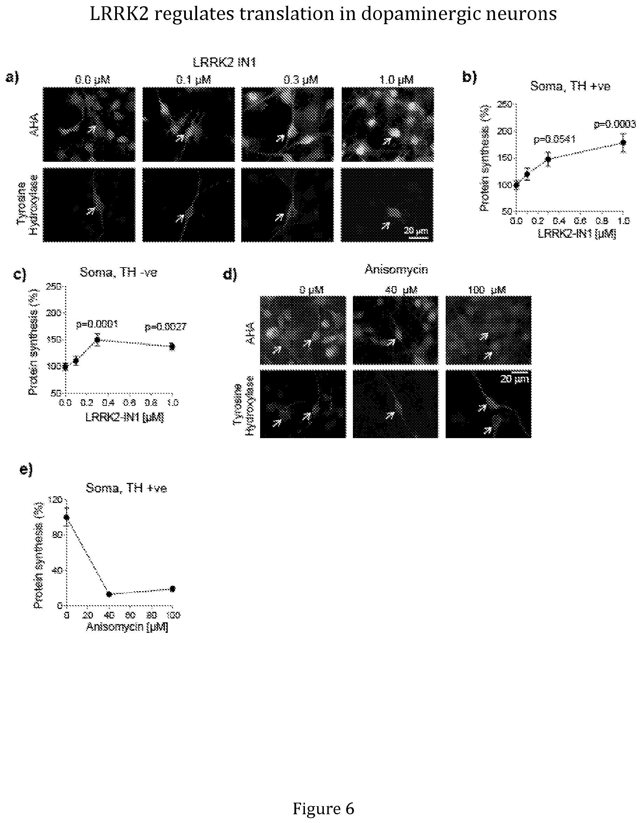

**Figure 6** uses click chemistry to label newly synthesised proteins with AHA. The findings show that inhibition of endogenous Lrrk2 increases protein translation in primary cultures of midbrain neurons. Dopaminergic neurons are visualised by tyrosine hydroxylase (TH) staining. a) Representative images of midbrain cultures treated with increasing concentrations of Lrrk2 IN2, and stained for TH is shown. b,c) protein translation in the somas of TH-positive and TH-negative cells is shown. d) The effect of anisomycin (to inhibit translation) on mid brain culture de novo protein synthesis is shown by representative images. e) Quantitative data from d is shown. p-values are shown on the graphs above the data points to which they apply.

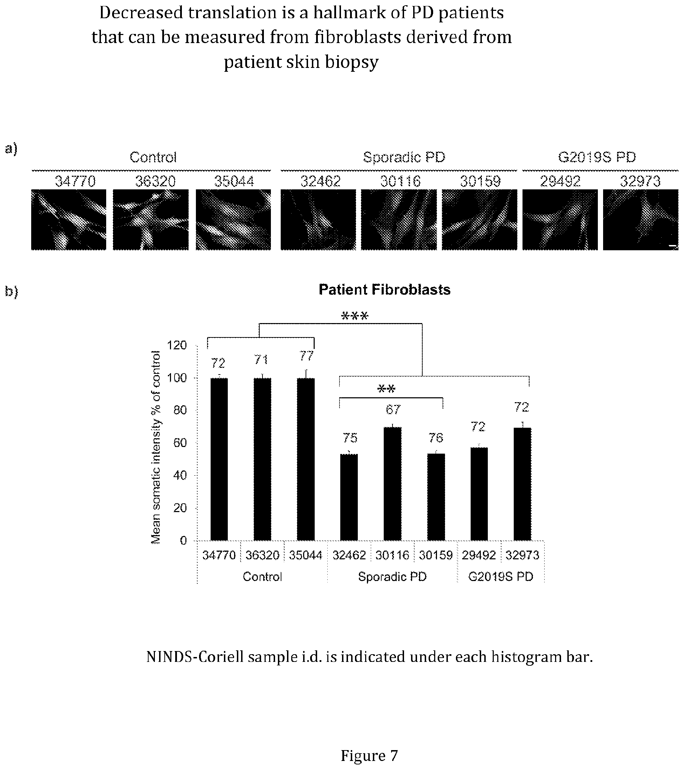

**Figure 7** shows that protein translation is significantly decreased in fibroblasts obtained from clinically diagnosed PD patients compared to agematched healthy controls. a) Representative fluorescence images of fibroblasts from healthy controls (Control) and individuals with sporadic PD or G2019S carriers. b) Quantitative data from patients is shown. Mean somatic intensity refers to the Alexa-488-alkyne fluorescence intensity and is a measure of protein translation. The individual's age is shown above the histogram bars. Standard errors of the mean are shown. **, p< 0.01; ***, p< 0.001.

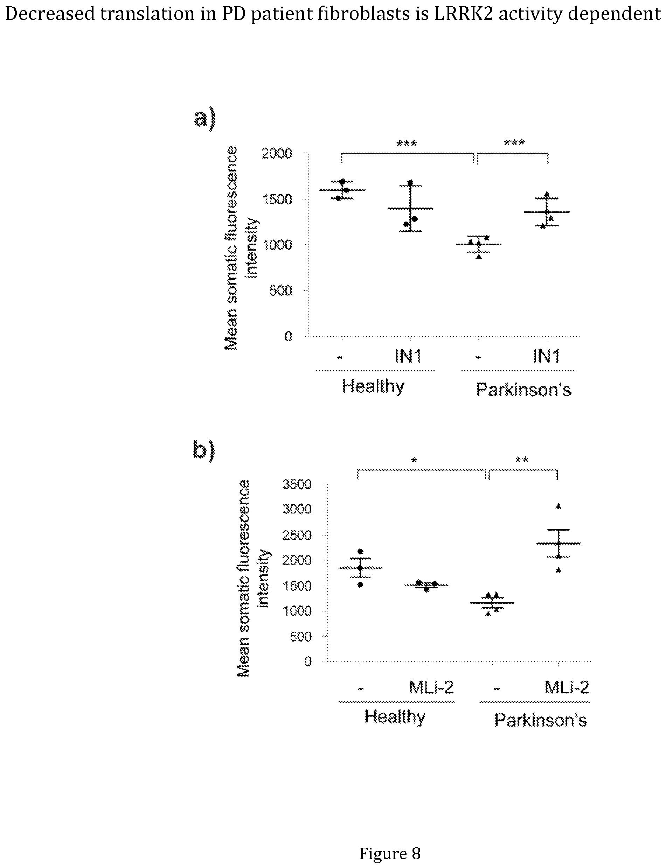

**Figure 8** shows the levels of protein synthesis measured from PD patient and healthy individual's cells. Protein synthesis levels are significantly decreased in the patient population. This decrease is reversed to a normal level in fibroblasts upon treatment with LRRK2 inhibitor-2 (IN2) or MLi-2. Patients were aged 67 to 75. Standard errors of the mean are shown. P-values, # p<0.0001; ***, p< 0.001.

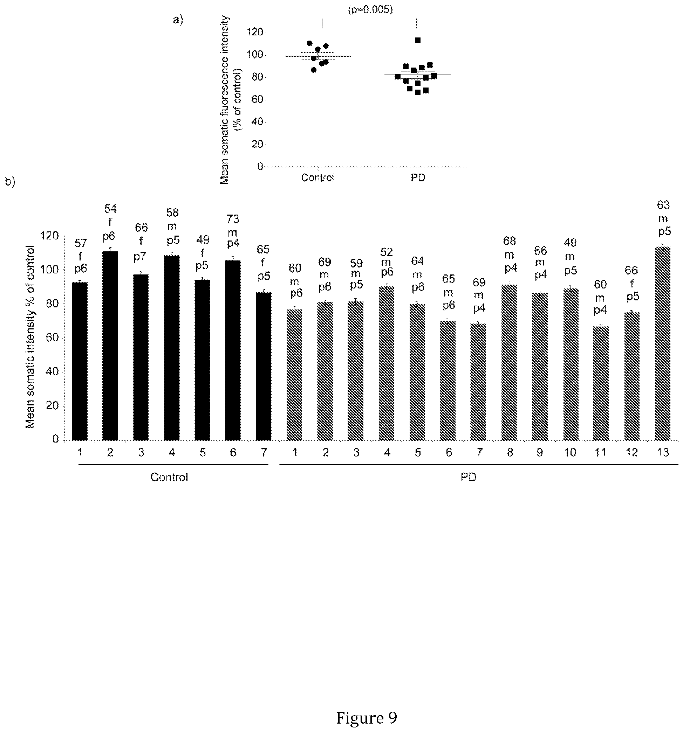

**Figure 9** shows protein synthesis levels in patient and healthy population from a separate cohort from Turku University Hospital in Finland. In this case, fibroblasts from individuals with probable PD (have motor symptoms but not yet diagnosed by DAT binding). a) Protein synthesis levels for control (healthy) and probable PD patients with mean and standard deviations is indicated. b) Individual data is shown with age, gender and cell passage number (pX) indicated above the histogram bars. Standard errors of the mean are shown. Control individuals (black, histogram bars 1 to 7), individuals with motor symptoms (grey, histogram bars 8 to 20). Most individuals shows a decrease in protein synthesis. One individual shows an increase.

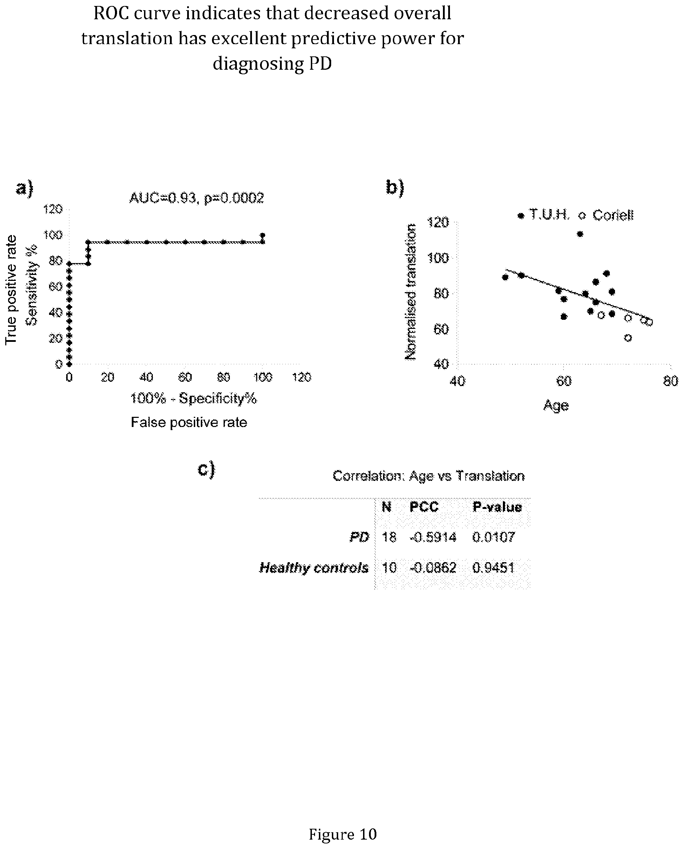

Figure 10 a) The receiver operating characteristic (ROC) curve for levels of mRNA translation in fibroblasts from patients and healthy individuals. This data combines all samples from two cohorts: (NINDs-Coriell) and the Turku University Hospital (T.U.H.). The area under the curve (AUC) was 93% indicating that levels of protein translation in the patient group provides good predictive power. Significance of AUC (p=0.0002) was determined using Student's t-test. The effect size was 1.86. (b) A Pearson's correlation analysis was carried out on the translation data from the same patients and controls. Protein translation versus age is shown for the T.U.H. cohort and NINDS-Coriell group. Decreased overall translation correlated negatively with age within the patient population. c) The Pearson's correlation coefficients (PCC) for PD patients and healthy controls are shown. In the healthy population, there is no significant correlation between decreased levels of translation and age indicating that decreased overall translation is a disease-specific hallmark of PD.

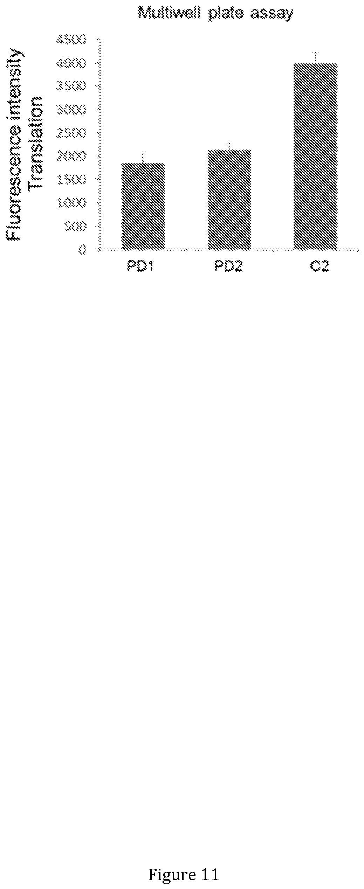

**Figure 11** shows that measurement of protein synthesis using fluorescence detection in a multi-well plate assay replicates findings from individual cell imaging. Patient fibroblasts (PD1, PD2) and healthy individual (C2) underwent L-azido-homoalanine (AHA) labelling in a 48-well plate followed by cycloaddition of Alexa fluor-488. Background fluorescence (i.e. fluorescence in the presence of 20 µM anisomycin) was subtracted. The multi-well assay replicated the decreased levels of translation observed in the same patients cells using fluorescence microscopy and individual cell analysis (Fig. 9). Standard errors of the mean are shown.

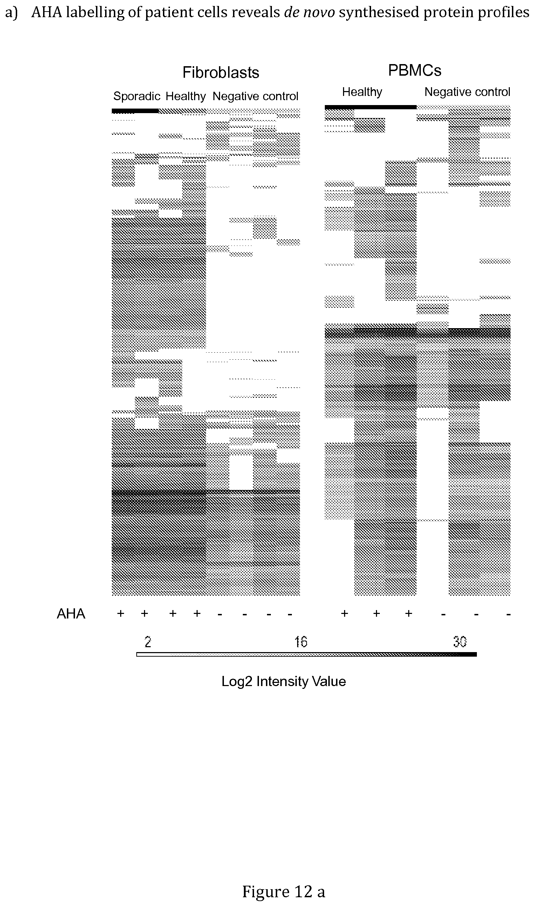

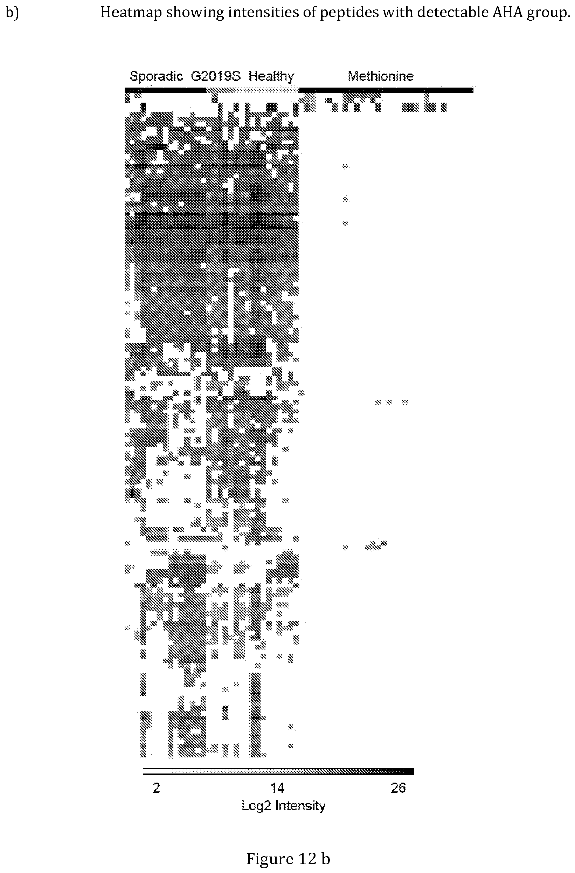

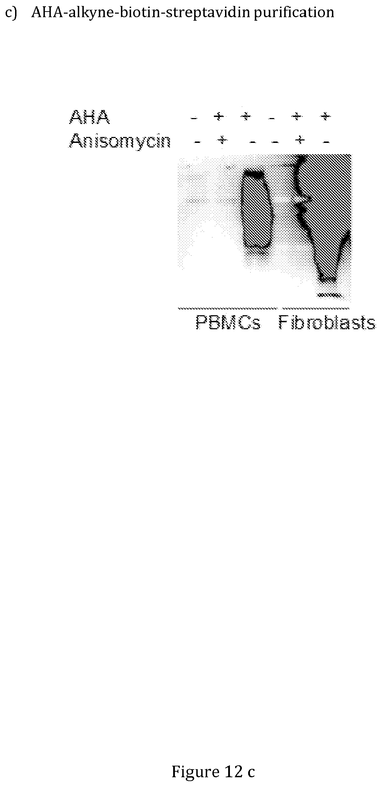

**Figure 12** shows the protein intensity measurements of de novo synthesised proteins in patient fibroblasts and peripheral blood mononuclear cells (PBMCs). Patient samples were from the NINDS-Coriell Institute in the USA, the European Biobank in Milan and the Turku University Hospital (T.U.H.) cohort in Finland. a) A heat map showing intensity values for MS/MS-identified nascent proteins from AHA-labelled cells (fibroblasts and PBMCs). Nascent proteins were isolated following covalent linkage to alkyne-agarose beads. After tryptic digestion, the released peptides were sequenced by mass spectrometry and identified using Maxquant software. As negative controls to determine non-specific protein binding, cells were labelled with methionine instead of AHA. These results show that AHA-alkyne bead enrichment works to isolate de novo synthesised proteins from patient cells. This approach yields a set of peptides that can be rapidly quantitated using a mass spectrometer using data dependant acquisition (DDA) using Selected Reaction Monitoring or Parallel Reaction Monitoring or by data independent acquisition (DIA) or using other types of mass spectrometry methods. An alternative is to use ELISA based detection with antibodies against the novel proteins in a singular or multiplexed fashion. b) The figure part shows the panel of L-azido-homoalanine (AHA)-labelled peptides where the AHA group was precisely identified using mass spectrometry. It is compared to samples that were labelled with methionine instead of AHA. This shows that the method successfully identifies de novo synthesised proteins labelled with AHA. c) Nascent proteins from AHA-labelled PBMCs and fibroblasts were purified by cyclo-addition to biotin-alkyne and purified using streptavidin beads. Immunoblotting with anti-biotin-HRP antibody was used to report protein translation levels. To test that the signal obtained was due to translation (and not non-significant binding to beads), samples treated with the translation inhibitor anisomycin were included. There was no signal in the presence of anisomycin, indicating the specificity of the labelling reaction for newly synthesised proteins.

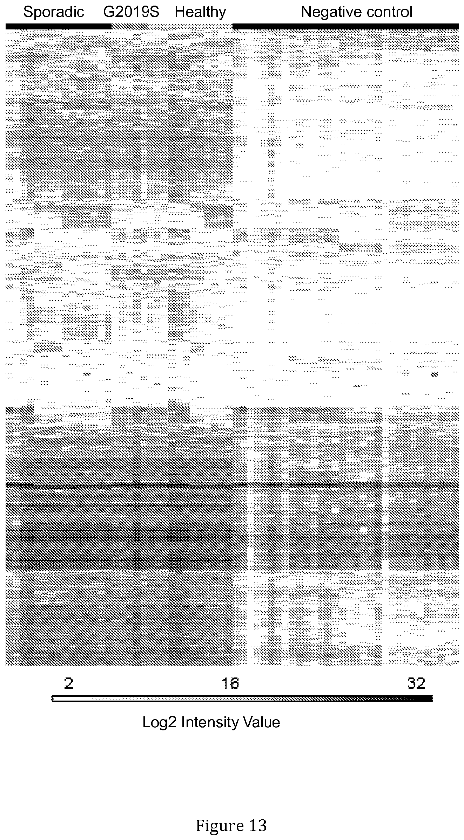

**Figure 13** shows the raw intensity values for patient and healthy individuals from 3 combined cohorts (NINDS, T.U.H. and European Biobank, Table 1). They are grouped according to diagnosis into sporadic, G2019S carriers and healthy. Negative controls are from the same fibroblasts labelled without AHA and represent non-specific background. Log2 intensity values are shown. In this data set, 1653 protein groups were identified. This illustrates the depth of information provided by analysis of *de novo* synthesised proteins. In all patient samples, levels of proteins were lower than in healthy individuals. For comparable MS/MS analysis protein levels were normalised before loading to HPLC. This will cause an underestimation of decreased overall translation in these samples. Even so, clear decreases can be seen. Also clear from this raw intensity data is the signature difference between sporadic, G2019S and healthy individuals.

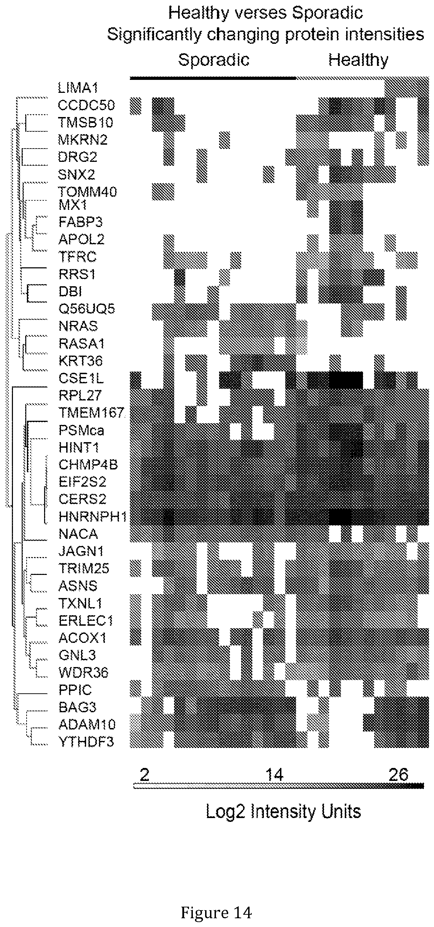

**Figure 14** shows a comparison of *de novo* synthesised proteins that are significantly altered in sporadic PD patients verses healthy individuals. Samples were combined from cohorts (NINDS-Coriell, T.U.H. and European Biobank). Protein names are shown on the y-axis and log2 protein intensity units are on the x-axis. Individual boxes represent different patients (left to right). Only protein levels that were significantly different between sporadic patients and healthy individuals (p-value <0.05) by Student's t-test are shown.

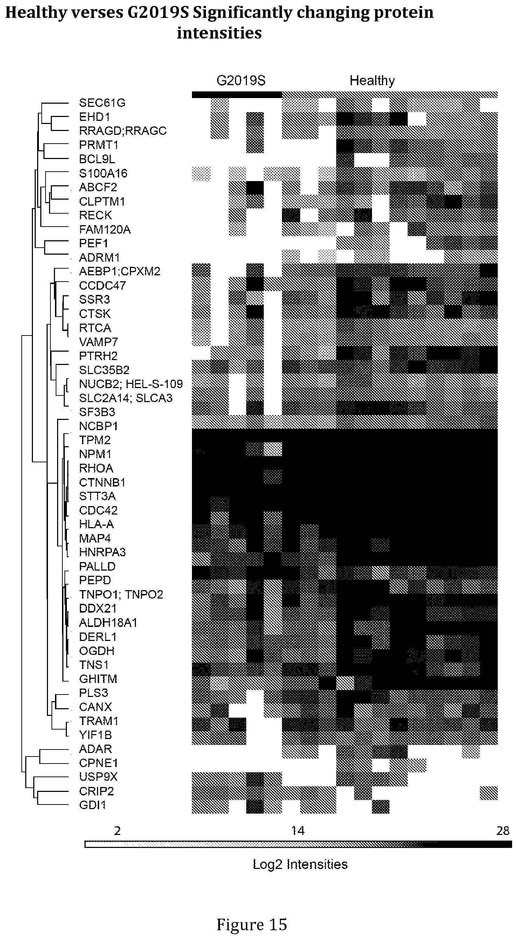

**Figure 15** shows a comparison of *de novo* synthesised proteins that were significantly different when comparing G2019S cases to healthy individuals. Samples were combined from cohorts (NINDS-Coriell, T.U.H. and European Biobank). Protein names are shown on the y-axis and log2 protein intensity units are on the x-axis. Individual boxes represent different patients (left to right).Only protein levels that were significantly different between G2019S patients and healthy individuals (p-value <0.05) by Student's t-test are shown.

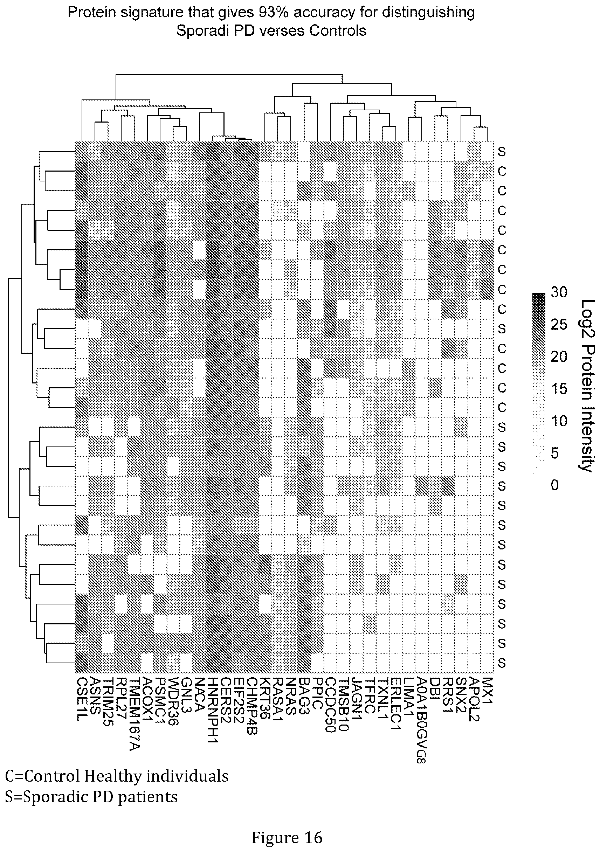

**Figure 16** shows a signature of 32 proteins where the intensity is significantly altered in sporadic PD compared to healthy individuals. This signature positively identified sporadic PD with a prediction accuracy of 93%. The patient data was combined from three separate patient groups in Finland, US and Italy. The Log2 protein intensity change relative to control is shown by grayscale LUT and in tables 2 and 3.

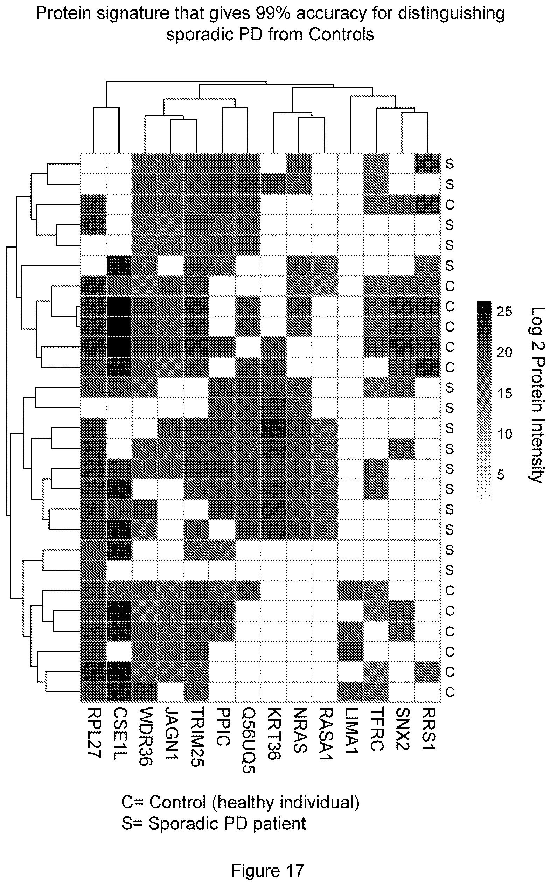

**Figure 17** shows a signature of 14 proteins where the intensity is significantly altered in sporadic PD compared to healthy individuals. This signature positively identified sporadic PD with a prediction accuracy of 99%. The patient data was combined from three separate patient cohorts in Finland, US and Italy. The Log 2 protein intensity change relative to control is shown by grayscale LUT and in tables 2 and 3.

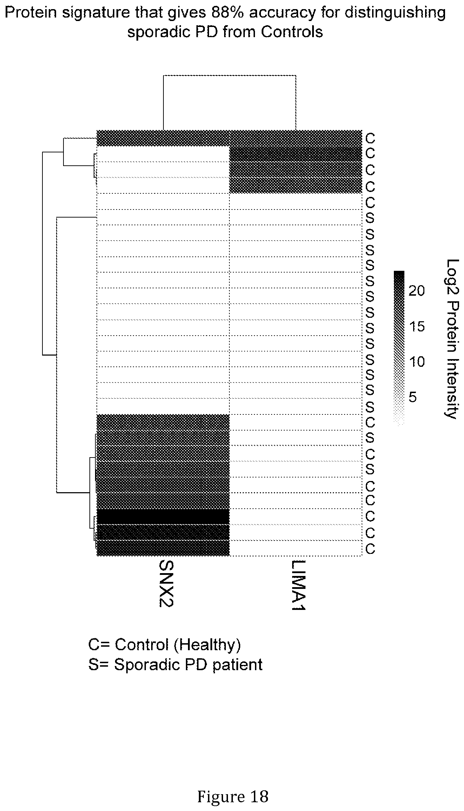

**Figure 18** shows a signature of 2 proteins where the intensity is significantly altered in sporadic PD compared to healthy individuals. These two proteins, SNX2 and LIMA1, positively identified sporadic PD with a prediction accuracy of 88%. The patient data was combined from three separate patient cohorts in Finland, US and Italy. The Log 2 protein intensity change relative to control is shown by grayscale LUT and in tables 2 and 3.

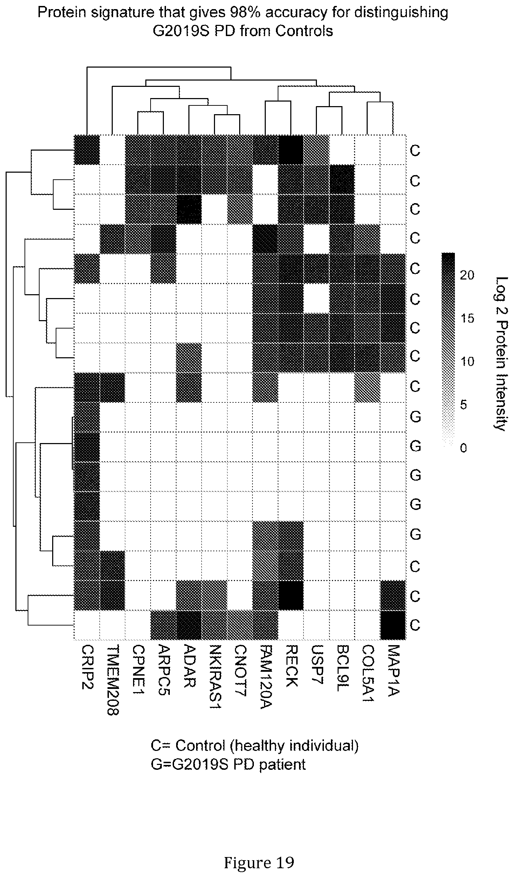

**Figure 19** shows a signature of 13 proteins where the intensity is significantly altered in G2019S PD compared to healthy individuals. This signature positively identified PD with a prediction accuracy of 98%. The patient data was combined from three separate patient cohorts in Finland, US and Italy. The Log 2 protein intensity change relative to control is shown by grayscale LUT and in tables 2 and 3.

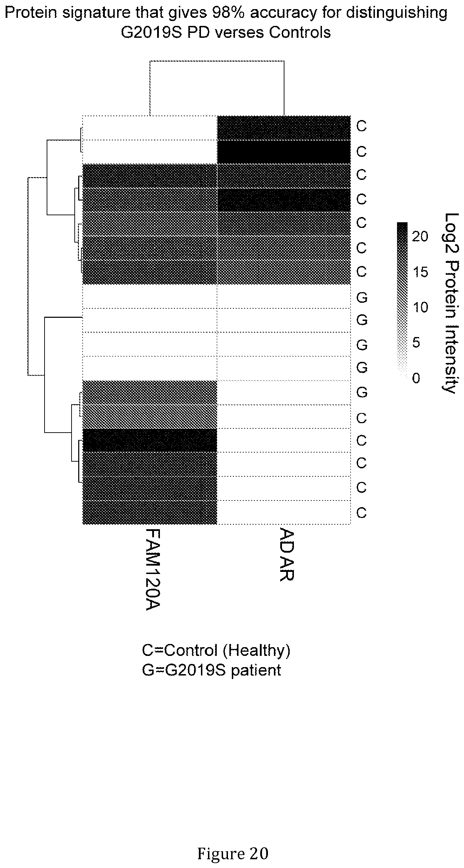

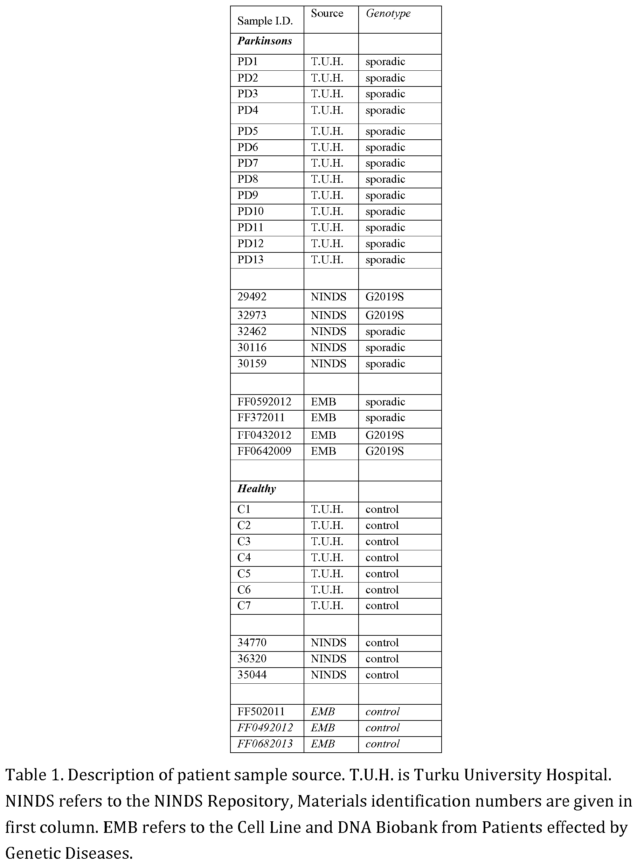

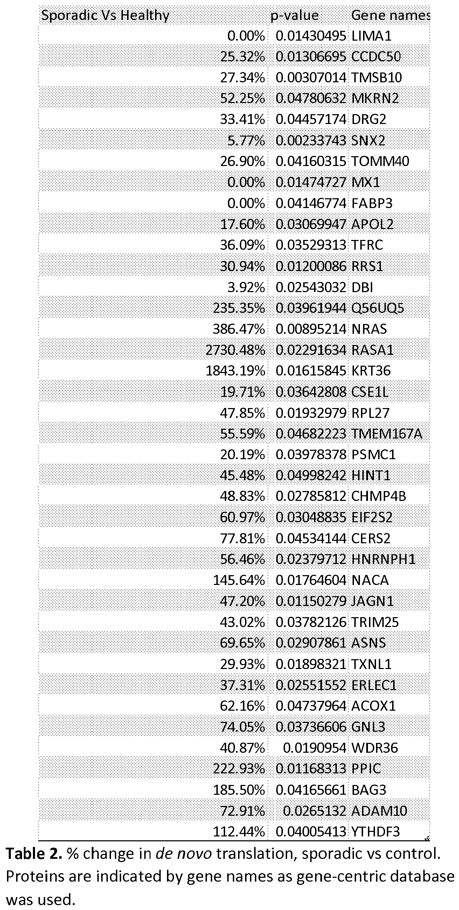

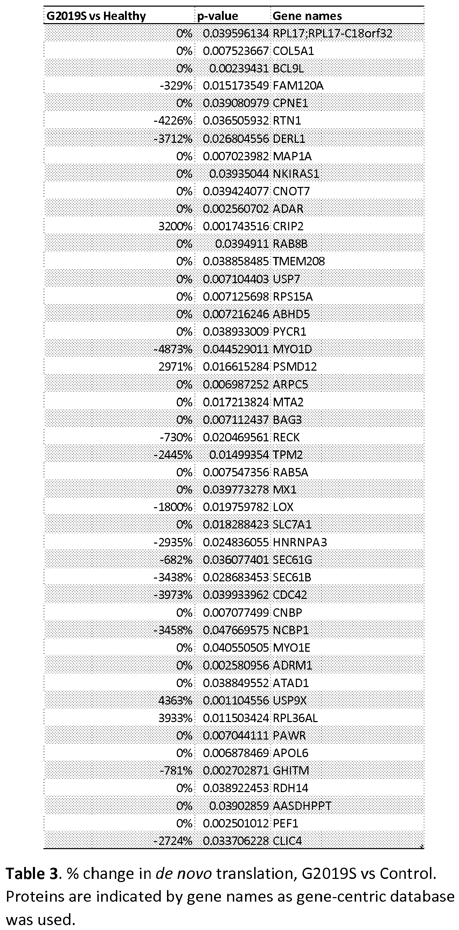

**Figure 20** shows a signature of 2 proteins where the intensity is significantly altered in G2019S PD compared to healthy individuals. These two proteins ADAR and FAM120A positively identified G2019S PD patients with a prediction accuracy of 98%. The patient data was combined from three separate patient cohorts in Finland, US and Italy. The Log 2 protein intensity change relative to control is shown by grayscale LUT and in tables 2 and 3.

**Table 1.** This table lists the patient material used in this study. The locally collected cohort from Turku University Hosptial (T.U.H) were used. We also acknowledge sharing of samples from the NINDS repository and the Cell line and DNA Biobank from Patients Affected by Genetic Diseases ⁵.

**Table 2** shows the change in protein intensity for sporadic PD patients. The indicated protein (defined by gene name) is described as a % of intensity from healthy individuals for the same protein. An equal match with healthy control would be shown as 100%. Patients are described in table 1.

**Table 3** shows the change in protein intensity for G2019S PD patients. The indicated protein (defined by gene name) is described as a % of intensity from healthy individuals for that protein. An equal match with healthy control would be 100%. Patients are described in table 1.

### DETAILED DESCRIPTION OF THE INVENTION

The present disclosure is based, at least in part, on studies aimed at elaborating the pathological mechanism of a gain of function mutation (G2019S) in leucine-rich repeat kinase 2 (LRRK2), a common cause of familial Parkinson's disease (PD). These studies have led to a surprising realization that PD is associated with decreased translation in both sporadic, non-inherited PD as well as in G2019S carriers. Thus, determination of decreased overall translation or of signatures resulting from decreased overall translation may be employed in diagnosing and monitoring both sporadic and inherited PD.

We set out to identify LRRK2-G2019S function in neurons with the expectation of learning more about sporadic PD. We found that LRRK2-G2019S phosphorylates ribosomal proteins (figure 1a and b). We wondered whether this could be relevant for PD and we therefore tested whether protein synthesis was altered in the rotenone model of PD (figure 2). Indeed we showed that this model of PD decreased overall translation could be measured with S35-metabolic labeling. We examined the rotenone model of PD further in neurons and identified that decreased overall translation was induced by rotenone when using azide-alkyne cyclo-addition with 488-Alexa fluorescent dye as a reporter of decreased overall translation (figure 3a-d). Moreover, we showed that rotenone-induced decreased translation was reversed under conditions when LRRK2 was inhibited, indicating a mechanistic link with PD. Also, in a reconstituted assay of translation where the hyperactive LRRK2-G2019S PD mutant protein was used decreased overall translation (figure 3e), further substantiating the link between PD and decreased translation. We further showed that rotenone decreased translation in dopaminergic neurons, the loss of which leads to the hallmark impaired motor function in PD. Also, dopaminergic neurons (tyrosine hydroxylase (TH)-positive cells) underwent neurite atrophy following rotenone treatment (figure 4). This was prevented by treatment with three structurally independent inhibitors of LRRK2 (IN1, GSK2578215A and MLi-1) (figure 4c-e). This showed that LRRK2 activation leading to decreased overall translation played a causal role in dying back of dopaminergic neuron neurites, a pathological hallmark of PD. Also, three structurally independent LRRK2 inhibitors significantly increased translation in healthy neurons, as measured using azide-alkyne cyclo-addition coupled to a Alexa-488 fluorescence readout (figure 5a-i, figure 6a-e). Together these results establish a link between elevated LRRK2 activity and decreased overall translation in cell and neuronal models of PD based on treatment with rotenone, an agent that is associated with PD in humans ⁶.

We next tested if decreased overall translation was a hallmark of PD that could be measured from patient samples. We measured translation in skin fibroblasts from healthy individuals, sporadic PD cases and G2019S PD cases, obtained from the Coriell Institute (NINDS). This showed that decreased overall translation, which is mechanistically linked to LRRK2 and PD pathology, could also be measured from peripheral cells obtained from skin biopsies from both sporadic non-inherited PD patients and from G2019S inherited PD patients alike (Figure 7). Figure 8 shows that decreased translation in patient skin cells is dependent on LRRK2. These data show that LRRK2 decreases translation both in sporadic PD and familial G2019S PD a like.

To test if decreased overall translation could provide a diagnostic readout from PD patients at an early stage of the disease, we recruited PD patients at Turku University Hospital in Finland. Thirteen volunteer patients with no family history of PD (termed sporadic) and seven healthy controls (agematched spouses or siblings) provided skin biopsies. We showed that overall translation could be measured from fibroblasts grown from these biopsies using azide-alkyne cyclo-addition chemistry as earlier (figures 3 to 8). This showed that overall decreased translation was a feature in early stage PD patient cells when compared to cells from healthy individuals (figure 9).

We next did a receiver operating characteristic (R.O.C.) analysis of patient data to determine the diagnostic potential of measuring decreased overall translation. For this, we combined the data obtained from the NINDS-Coriell patient group with the T.U.H. cohort (Table 1). This yielded a size effect of 1.86 and area under the curve (A.U.C.) of 93% (figure 10a). This indicates that measuring overall decreased translation has excellent discriminating capacity to separate PD patients from healthy controls using cyclo-addition chemistry and fluorescence reporter as described in examples 3 to 8. We next showed that decreased overall translation correlated with age in the patient population (figure 10b). Thus patients that were older or had symptoms for longer showed a larger decrease in overall translation. Note, no negative correlation with age was found in healthy individuals (Figure 10c). This means that decreased overall translation is not a feature of normal ageing but is a disease-specific hallmark of PD. These results show that the readout of decreased overall translation is likely to be useful for measuring disease progression and therefore holds value for patient monitoring during treatment as well as for new drug evaluation during clinical trials or for drug repurposing, where the drugs are related to the alleviation of PD symptoms or halting disease progression.

We next showed that decreased overall translation could also be measured using a spectrophotometer-based multi-well assay (figure 11). This yielded equivalent results to the method used in examples 7 to 8 where fluorescent microscopy was used. The multi-well assay provides an alternative analysis method that can be used in high-throughput mode to form the basis of a diagnostic kit to measure decreased translation as a readout that defines PD.

Accordingly, in some embodiments the present invention provides a method of diagnosing or monitoring PD in a subject on the basis of decreased overall translation, said decreased overall translation being indicative of PD. The method may be carried out by using any suitable technique available in the art, including but not limited to the techniques mentioned above. Also provided is a kit for the practice of such a method.

We next showed that protein signatures of translational output could be identified and used as a readout of decreased overall translation. These signatures could distinguish PD from healthy individuals. For this, we first showed that isolation of *de novo* synthesized proteins from AHA-labelled cells using alkynetagged agarose could be used to separate newly translated proteins. This yielded purified samples of newly synthesized proteins from patient cells that could be sequenced using mass spectrometry and the intensity of each protein when compared to samples from healthy controls provided a detailed signature of decreased overall translation in PD. We first demonstrated that this method faithfully identified newly translated proteins in PD patients with little background interference (figure 12a). By restricting the MS/MS search to only those peptides that contained AHA groups, we were able to show the method works efficiently to enrich for *de novo* synthesized proteins that provided a detailed fingerprint or signature of decreased overall translation (figure 12b). Finally, we showed that decreased overall translation could also be measured using an antibody-based method (figure 12c).

We went on to measure MS/MS signatures of decreased overall translation from groups of sporadic PD and G2019S cases compared to healthy controls (Figures 13-15). These patient groups included the T.U.H. early stage PD cohort and the NINDS-Coriell cohort as before but in addition, it included a group of patient samples from the European Biobank. All patient samples are described in Table 1. We identified 39 *de novo* translated proteins that were significantly changed in intensity (either increased or decreased) in sporadic PD compared to healthy controls (figure 14). 80% of these protein showed significantly decreased intensity. Group 1 proteins, those characterized by decreased intensities acquired by HPLC-MS/MS were (by gene name) LIMA1, CCDC50, TMSB10, MKRN2, DRG2, SNX2, TOMM40, MX1, FABP3, APOL2, TFRC, RRS1, DBI, CSE1L, RPL27, TMEM167, PSMca, HINT1, CHMP4B, EIF2S2, CERS2, HNRNPH1, JAGN1, TRIM25, ASNS, TXNL1m ERLEC1, ACOX1, GNL3, WDR36, and ADAM10. Group 2 proteins, the 20% with significantly increased intensities were (by gene name) Q56UQ5, NRAS, RASA1, KRT36, PPIC, BAG3 and YTHDF3. Measurement of group 1 (decreasing) or group 2 (increasing) proteins by mass spectrometry as shown here (figure 14), or by other methods such as antibody-based, provides a basis for determining decreased overall translation as a readout that is diagnostic of sporadic PD.

Accordingly, in some embodiments the present invention provides a method of diagnosing and monitoring PD, said method comprising testing or assaying a sample from a subject suspected of suffering from PD for the presence of altered gene expression of one or more genes selected from the group consisting of LIMA1, CCDC50, TMSB10, MKRN2, DRG2, SNX2, TOMM40, MX1, FABP3, APOL2, TFRC, RRS1, DBI, CSE1L, RPL27, TMEM167, PSMca, HINT1, CHMP4B, EIF2S2, CERS2, HNRNPH1, JAGN1, TRIM25, ASNS, TXNL1m ERLEC1, ACOX1, GNL3, WDR36, ADAM10. Q56UQ5, NRAS, RASA1, KRT36, PPIC, BAG3 and YTHDF3, or any combination thereof. In more specific embodiments, decreased expression of one or more genes selected from the group consisting of LIMA1, CCDC50, TMSB10, MKRN2, DRG2, SNX2, TOMM40, MX1, FABP3, APOL2, TFRC, RRS1, DBI, CSE1L, RPL27, TMEM167, PSMca, HINT1, CHMP4B, EIF2S2, CERS2, HNRNPH1, JAGN1, TRIM25, ASNS, TXNL1m ERLEC1, ACOX1, GNL3, WDR36, and ADAM10, or any combination thereof, and/or increased expression of one or more genes selected from the group consisting of Q56UQ5, NRAS, RASA1, KRT36, PPIC, BAG3 and YTHDF3, or any combination thereof, is indicative of PD, sporadic PD in particular. In some preferred embodiments, the sample to be tested or assayed comprises fibroblasts. The method may be carried out by using any suitable technique available in the art, including but not limited to the techniques mentioned above.

We next identified 56 *de novo* translated protein intensities that were significantly altered in G2019S patients compared to healthy controls. Among these, 55 out of 56 proteins were significantly decreased in intensity in G2019S cases compared to control. These were (by gene name) GDI1, USP9X, CRIP2, ADRM1, FAM120A, BCL9L, PEF1, ADAR, YIF1B, CANX, GHITM, CANX, TNS1, OGDH, TNPO1 and 2, PEPD, HNRNFA3, MAP4, ALDH18A1, DDX21, DERL1, TPM2, NPM1, CLIC4, HLA-A, CDC41, STT3A, RHOA, CTNNB1, TRAM1, PLS3, SSR3, CTSK, NCBP1, PTRH2, SLC35B2, SF3B3, SLC2A14, SLC2A3, NUCB2; HEL-S-109, SEC61G, CLPTM1, RECK, PRMT1, ABCF2, VAMP7, RTCA, AEBP1, CPXM2, S100A16, RRAGD, RRAGC, EHD1. The one protein that increased in intensity was (by gene name) PALLD. Measurement of decreasing proteins or increased levels of PALLD by mass spectrometry as shown here (figure 15), or by other methods such as antibody-based, provides a basis for determining decreased overall translation as a readout that is diagnostic of PD. This readout is relevant for familial PD, especially G2019S carriers. It could also provide a readout of decreased overall translation in a subgroup of sporadic PD patients, yet to be defined, and in this way could be used for patient stratification. Significantly, all patient and control samples underwent normalization for protein concentration prior to HPLC-MS/MS, as a requisite preparation step.

Accordingly, in some further embodiments the present invention provides a method of diagnosing and monitoring PD, said method comprising testing or assaying a sample from a subject suspected of suffering from PD for the presence of altered gene expression of one or more genes selected from the group consisting of GDI1, USP9X, CRIP2, ADRM1, FAM120A, BCL9L, PEF1, ADAR, YIF1B, CANX, GHITM, CANX, TNS1, OGDH, TNPO1 and 2, PEPD, HNRNFA3, MAP4, ALDH18A1, DDX21, DERL1, TPM2, NPM1, CLIC4, HLA-A, CDC41, STT3A, RHOA, CTNNB1, TRAM1, PLS3, SSR3, CTSK, NCBP1, PTRH2, SLC35B2, SF3B3, SLC2A14, SLC2A3, NUCB2; HEL-S-109, SEC61G, CLPTM1, RECK, PRMT1, ABCF2, VAMP7, RTCA, AEBP1, CPXM2, S100A16, RRAGD, RRAGC, EHD1 and PALLD, or any combination thereof. In more specific embodiments, decreased expression of one or more genes selected from the group consisting of GDI1, USP9X, CRIP2, ADRM1, FAM120A, BCL9L, PEF1, ADAR, YIF1B, CANX, GHITM, CANX, TNS1, OGDH, TNPO1 and 2, PEPD, HNRNFA3, MAP4, ALDH18A1, DDX21, DERL1, TPM2, NPM1, CLIC4, HLA-A, CDC41, STT3A, RHOA, CTNNB1, TRAM1, PLS3, SSR3, CTSK, NCBP1, PTRH2, SLC35B2, SF3B3, SLC2A14, SLC2A3, NUCB2; HEL-S-109, SEC61G, CLPTM1, RECK, PRMT1, ABCF2, VAMP7, RTCA, AEBP1, CPXM2, S100A16, RRAGD, RRAGC, and EHD1, or any combination thereof, and/or increased expression of PALLD is indicative of PD, familial PD in particular. In some preferred embodiments, the sample to be tested or assayed comprises fibroblasts. The method may be carried out by using any suitable technique available in the art, including but not limited to the techniques mentioned above.

In a clinical diagnostics setting where signatures are used, a smaller number of features can have advantages over signatures with more features. We therefore applied machine learning to reduce the number of disease defining features in signatures for sporadic and G2019S PD. By reducing the number of features in the panel for sporadic PD from 39 to 32 we obtained a prediction accuracy of 93%. This signature is shown in figure 16. Decreased levels of the following proteins (described by gene name) MX1, APOL2, SNX2, RRS1, DBI, AOA1BOGVG8, LIMA1, ERLEC1, TSNL1, TFRC, JAGN1, TMSB10, CCDC50, CHMBP4B, EIF2S2, CERS2, HNRNPH1, NACA, GNL3, WDR36, PSMC1, ACOX1, TMEM167A, RPL27, TRIM25, ASNS and CSE1L can be used to demonstrate decreased overall translation as a means to diagnose sporadic PD, while increased levels of NRAS, RASA1, KRT36, PPIC or BAG3 contribute predictive power.

Accordingly, in some further embodiments the present invention provides a method of diagnosing and monitoring PD, said method comprising testing or assaying a sample from a subject suspected of suffering from PD for the presence of altered gene expression of one or more genes selected from the group consisting of MX1, APOL2, SNX2, RRS1, DBI, AOA1BOGVG8, LIMA1, ERLEC1, TSNL1, TFRC, JAGN1, TMSB10, CCDC50, CHMBP4B, EIF2S2, CERS2, HNRNPH1, NACA, GNL3, WDR36, PSMC1, ACOX1, TMEM167A, RPL27, TRIM25, ASNS, CSE1L, NRAS, RASA1, KRT36, PPIC or BAG3, or any combination thereof. In more specific embodiments, decreased expression of one or more genes selected from the group consisting of MX1, APOL2, SNX2, RRS1, DBI, AOA1BOGVG8, LIMA1, ERLEC1, TSNL1, TFRC, JAGN1, TMSB10, CCDC50, CHMBP4B, EIF2S2, CERS2, HNRNPH1, NACA, GNL3, WDR36, PSMC1, ACOX1, TMEM167A, RPL27, TRIM25, ASNS and CSE1L, or any combination thereof, and/or increased expression of one or more genes selected from the group consisting of NRAS, RASA1, KRT36, PPIC and BAG3, or any combination thereof, is indicative of PD, sporadic PD in particular. In some preferred embodiments, the sample to be tested or assayed comprises fibroblasts.

We next imposed a threshold cut-off of two-fold change (increase or decrease compared to control) and did further training of the model and reduced the number of features to 14. This signature contained RRS1, RASA1, KRT36, Q56UQ5, PPIC and NRAS which increased in intensity in sporadic PD and SNX2, TFRC, LIMA1, TRIM25, JAGN1, WDR36, CSE1L and RPL27 which decreased in intensity compared to controls and has a prediction accuracy of 98% for distinguishing sporadic PD from healthy individuals (figure 17). We used *brut force* pairwise comparison of protein signatures for sporadic prediction. The resulting paired signature was SNX2 and LIMA1 with a prediction accuracy of 88%. Decreased levels of SNX2 was the protein that alone could distinguish sporadic PD from healthy controls. Therefore, the signatures in figure 17 and 18 (when intensity levels differ from control by greater than two fold) or decreased levels of SNX2 alone, can be used to define decreased overall translation as a diagnosis method for sporadic PD.

Accordingly, in some further embodiments the present invention provides a method of diagnosing and monitoring PD, said method comprising testing or assaying a sample from a subject suspected of suffering from PD for the presence of altered gene expression of one or more genes selected from the group consisting of RRS1, RASA1, KRT36, Q56UQ5, PPIC, NRAS, SNX2, TFRC, LIMA1, TRIM25, JAGN1, WDR36, CSE1L and RPL27, or any combination thereof. In some more specific embodiments, decreased expression of one or more genes selected from the group consisting of SNX2, TFRC, LIMA1, TRIM25, JAGN1, WDR36, CSE1L and RPL27, or any combination thereof, and/or increased expression of one or more genes selected from the group consisting of RRS1, RASA1, KRT36, Q56UQ5, PPIC and NRAS, or any combination thereof, is indicative of PD, sporadic PD in particular. Preferably, the method may be based on decreased levels of SNX2 and LIMA1, or even decreased levels of SNX2 only. In some preferred embodiments, the sample to be tested or assayed comprises fibroblasts.

We next used model training to reduce the number of features in a signature readout for G2019S PD. Only proteins that changed by two-fold compared to control were included. This gave a signature with 16 features and prediction accuracy of 98% (figure 19). This included proteins that decreased in intensity compared to control MAP1A, COL5A1, BCL9L, USP7, RECK, FAM120A, CNOT7, NKIRAS1, ADAR, ARPC5, CPNE1, TMEM208 and one protein that increased in intensity, CRIP2. We used *brut force* pair wise comparison to find the best pair of disease defining proteins for G2019S PD. This identified FAM120A and ADAR as the best pair with a prediction accuracy of 98%.

Accordingly, in some further embodiments the present invention provides a method of diagnosing and monitoring PD, said method comprising testing or assaying a sample from a subject suspected of suffering from PD for the presence of altered gene expression of one or more genes selected from the group consisting of MAP1A, COL5A1, BCL9L, USP7, RECK, FAM120A, CNOT7, NKIRAS1, ADAR, ARPC5, CPNE1, TMEM208 and CRIP2, or any combination thereof. In some more specific embodiments, decreased expression of one or more genes selected from the group consisting of MAP1A, COL5A1, BCL9L, USP7, RECK, FAM120A, CNOT7, NKIRAS1, ADAR, ARPC5, CPNE1, and TMEM208, or any combination thereof, and/or increased expression of CRIP2 is indicative of PD, G2019S PD in particular. Preferably, the method may be based on decreased levels of FAM120A and ADAR. In some preferred embodiments, the sample to be tested or assayed comprises fibroblasts.

The signatures in figures 14 and 15 list protein intensity changes of significantly altered translation (as defined above) in sporadic (figure 14) and G2019S (figure 15) PD. The signatures in figures 16, 17 and 18 list proteins the intensity of which defines sporadic PD. The signatures in figures 19 and 20 list proteins the intensity of which defines G2019S PD. In the future, these signatures can be applied within the same models to diagnose patient samples, without the need for new control samples. The control levels have already been set by the model. Alternatively these signatures can be further trained when new patient or control samples are available so that the prediction is improved even further. We have tested our linear regression model by dividing samples into two subgroups; a training set that consists of only definitively diagnosed PD (DAT binding deficiency) cases verses healthy controls and a group on which the trained model is tested. The results were compliant to a high level with neurological diagnosis. This indicates that the model, in its current state, trained on data from three independent sites (US, Finland and Italy), provides a valuable test to assist PD patient diagnosis.

The signatures described in examples 10 to 14 were identified based on measurement of *de novo* translated proteins. However their use is not restricted to analysis of such samples. These signatures can equally be used for measurements from crude cell lysates, tissue biopsies or body fluids. Clearly if overall translation is decreased in PD, then a simple measurement from crude samples (as described above) will also be indicative of decreased translation. For such an embodiment, mass spectrometry assays such as SRM, PRM, MRM, DDA or DIA would be suitable. These methods or antibody methods could be used to detect these signatures, or new combinations of proteins from these lists to generate new signatures, or single proteins from these lists can be used to define decreased overall translation as a readout of PD.

The examples (10 to 14) herein demonstrate that sporadic and G2019S PD can be characterized by distinct signatures of newly translated proteins. This can represent differences in the underlying pathological mechanism for these two patient groups, as PD is a heterogenous disorder. These profiles can be used to distinguish between sporadic from G2019S PD. In this way these signatures can be used to stratify patients for disease monitoring or for defining custom treatments. These signatures can also be useful to define further sub-types of PD. As an example of this, hierarchical clustering reveals several further stratification within patient groups (figures 14 to 20).

Measuring decreased overall translation as an indication of PD is a diagnostic test that has mechanistic basis in the disease process itself as shown by examples 1 to 8. This is in contrast to biomarker assays that rely on *"guilt by association"* logic. Biomarker assays for PD based are often proposed based on genetic screening. However as genetic forms of PD account for only a small percentage of cases (~5%), in a clinical setting, genetic testing for PD is not a part of the diagnostic process unless a family history of PD is known ⁴. Thus for example diagnosis of PD by screening for LRRK2-G2019S mutation has been shown only to be useful for assisting diagnosis in symptomatic patients with a family history of PD ⁷. Our readout based on overall decreased translation is not restricted to this subgroup of patients, as demonstrated by examples. It works well for defining disease in both sporadic PD cases and LRRK2-G2019S cases. Interestingly, manifestations of this readout, ie signatures in figures 16 to 10, show that these patient groups have molecularly distinct translation profiles.

As used herein, the term "indicative of PD", when applied to decreased overall translation, refers to a level of translation which, using routine statistical methods setting confidence levels at a minimum of 95%, is diagnostic of PD or a stage of PD such that the detected level of translation is found significantly more often in subjects with PD or a stage of PD than in subjects without PD or another stage of PD. Preferably, a level of translation which is indicative of PD is found in at least 80% of subjects who have the disease and is found in less than 10% of subjects who do not have the disease. More preferably, a level of translation which is indicative of PD is found in at least 90%, at least 95%, at least 98%, or more in subjects who have the disease and is found in less than 10%, less than 8%, less than 5%, less than 2.5%, or less than 1% of subjects who do not have the disease.

Accordingly, the term "decreased overall translation" may refer to quantitatively altered protein synthesis as compared to that in a relevant control sample. In some embodiments said decreased overall translation refers to impaired translation. As used herein, the terms "translation" and "protein synthesis" are interchangeable. Alternatively, a set of signature proteins or peptides that are unique to the disease state may be used in a qualitative analysis of decreased overall translation.

As used herein, the term "impaired translation" refers to a decrease or reduction in the level of translation in a sample as compared with that of a relevant control sample. It may also involve the production of novel protein or peptides or cause the use of alternative reading frames. Said decrease can be determined using standard methods known in the art. The translation is decreased if the level of translation in the sample is, for instance, at least about 1.5 times, 1.75 times, 2 times, 3 times, 4 times, 5 times, 6 times, 8 times, 9 times, time times, 10 times, 20 times or 30 times lower than the level of translation in the control sample. In some embodiments, the term "impaired translation" refers to a statistically significant decrease in the level of translation as compared with that of a relevant control. In a preferred embodiment fibroblasts and blood samples as well as other bodily fluids including but not limited to urine, saliva and cerebral spinal fluid, can be used to measure impaired translation.

Quantitative assessment of translation refers to the determination of the amount of proteins that a cell synthetizes, or to the determination of the level of translation in a cell, and it may be directed to the totality of proteins synthetized by a cell, or to any appropriate sub-fraction thereof, such as those that can be defined as diagnostic signatures. The terms "level" and "amount" can refer to an absolute or relative quantity.

The level of translation in a biological sample may be determined by a variety of techniques as is readily apparent to those skilled in the art. For instance, metabolic labelling may be used for incorporating molecules into *de novo* synthesized proteins by endogenous synthesis and modification machinery of living cells. This typically is carried out by culturing cells in the presence of an amino acid that is either directly detected by virtue of its radioactive decay (e.g. S35-methionine) or that can be detected by covalent linkage to a tag. Non-limiting examples of suitable labels for such purposes include fluorescent labels such as Alexa dyes (e.g. Fig. 3, 4, 6, 7, 8, 9, 10, 11) for quantitative fluorescence detection methods such as microscopy or spectroscopy and other bio-conjugate techniques.

Alternatively, click chemistry may be employed for determining the presence or absence of decreased overall translation in a cell. In some embodiments, click chemistry may be based on biosynthetic incorporation of azide- or alkylene-bearing methionine analogues such as L-azido-homoalanine (AHA) or homopropargylglycine (HPG). The resulting azide- or alkylene-labelled proteins can be detected by copper-I-catalyzed alkyne-azide cycloaddition (CuAAC) with reagents for fluorescent detection (e.g. Fig. 3, 4, 6, 7, 8, 9, 10, 11) or affinity purification using alkyne-agarose and identification by mass spectrometry (e.g. Fig. 12, 13, 14, 15, 16, 17, 18, 19, 20) or using alkyne-biotin-neutravidin-sepharose conjugates and detection using anti-biotin antibodies or mass spectrometry (e.g. Fig. 12). Visualization of incorporated AHA may also be carried out e.g. by conventional fluorescence microscopy using an approach called FUNCAT (Fluorescent Non-Canonical Amino acid Tagging).

Additionally, antibodies could be utilised to identify aberrantly translated proteins or the use of inhibitors of translation initiation (e.g. harringtonine) or translocation (e.g. cycloheximide) could be employed to enrich for N-terminal peptides of nascent proteins of interest.

In some embodiments, decreased overall translation may also be determined qualitatively on the basis of signature molecules indicative of decreased overall translation resulting from the use of alternative open reading frames or changes in post-translational modification, or signatures indicative of decreased overall translation even though the precise mechanism of translation block is not known. Suitable methods for measuring signatures could include mass spectrometry selected reaction monitoring (SRM) assays, multiple reaction monitoring (MRM) or parallel reaction monitoring (PRM) assays of translation products (as well as DDA shotgun or DIA approaches) that provide diagnostic or predictive information related to the disease. Additionally, antibody-based methods for example ELISA, for detection of decreased overall translation could be used to measure a single protein or group of proteins designating a "signature" that results from decreased overall translation and is characteristic of or predictive of disease state.

To determine whether a level of translation is indicative of a disease, the translation level in a relevant control has to be determined. Once the control levels are known, the determined translation levels can be compared therewith and the significance of the difference can be assessed using standard statistical methods. In some embodiments of the present disclosure, a statistically significant difference between the determined translation level and the control level is indicative of PD. In some further embodiments, before to be compared with the control, the translation levels are normalized using standard methods.

In the case of the production of novel protein isoforms, the method can be used without reference to a control sample. The detection methods envisioned include detection by mass spectrometry, ELISA type antibody recognition or by measuring the enzymatic activity of unique translation products.

Likewise, to determine whether a given molecule signature is indicative of a disease, a corresponding signature in a relevant control has to be determined. Once the control signature is known, the determined signature can be compared therewith. Differences between said signatures may be indicative of the disease. However, unique signatures may also be used as indications of decreased overall translation in a non-comparative or absolute manner, i.e. even without comparisons with control signatures.

As used herein, the term "control" may refer to a control sample obtained from an apparently healthy individual or a pool of apparently healthy individuals, or it may refer to a predetermined threshold value or a molecular signature which is indicative of the presence or absence of PD. Statistical methods for determining appropriate threshold values will be readily apparent to those of ordinary skill in the art. The control or threshold values, as well as control signatures, may have been determined, if necessary, from samples of subjects of the same age, demographic features, and/or disease status, etc. The control or threshold value, or the control signature may originate from a single individual not affected by PD or be a value pooled from more than one such individual.

As used herein, the term "apparently healthy" refers to an individual or a pool of individuals who show no signs of PD and thus are believed not to be affected by PD and/or who are predicted not to develop PD.

As used herein, the term "subject" refers to an animal, preferably to a mammal, more preferably to a human. Depending on the embodiment in question, said subject may suffer from PD with or without diagnosis, be suspected to suffer from PD, be at risk of PD, or may have already been treated for PD. Herein, the terms "human subject", "patient" and "individual" are interchangeable.

As used herein, the term "sample" refers to a biological sample, typically a clinical sample which may be, for example, a tissue sample such a skin sample, a cell sample such as a skin cell sample (e.g. a skin fibroblast sample), or a skin punch or skin shave, or a sample of a bodily fluid such as urine, blood, plasma, or serum, obtained from a subject. Generally, obtaining the sample to be analysed from a subject is not part of the present methods, which may therefore be regarded as *in vitro* methods. Preferred samples include fibroblasts from a skin biopsy or the biopsy itself, or from peripheral mononuclear blood cells from a blood sample or from exosomes isolated from blood. Consequently, methods of the present invention may be carried out in a minimally invasive fashion.

The present invention can be put to practice in different ways. First, decreased overall translation may be used for diagnosing PD. As used herein, the term "diagnosing" refers, without limitation, to a process aimed at determining whether or not a subject is afflicted with PD. This is also meant to include instances where the presence or a stage of PD is not finally determined but that further diagnostic testing is warranted. In such embodiments, the method is not by itself determinative of the presence or absence of PD, or the stage of PD in the subject but can indicate that further diagnostic testing is needed or would be beneficial. Therefore, the present method may be combined with one or more other diagnostic methods for the final determination of the presence or absence of PD, or the stage of PD in the subject. Such other diagnostic methods are well known to those skilled in the art, and include but are not limited to, neurological examinations and brain scans such as Positron Emission Tomography (PET) or single photon emission computed tomography (SPECT) scan with [¹²⁵I]FP-CIT and mass spectrometry. Mass spectrometry may be used to detect changes in the absolute or relative expression level(s) of a protein or set of proteins (or their peptide products produced by digestion). This is to be taken to include changes in alternative splicing of proteins, their Post-Translational Modifications or changes in the structure of the post-translational modifications themselves (e.g. changes in glycan structures of O- or N-linked glycosylated peptides).

Importantly, the present invention enables early detection or diagnosis of PD, preferably at the prodromal stage, or prior to any significant loss of dopaminergic neurons. Early diagnosis of PD could allow early treatment and, thus, delay progression. Moreover, early diagnosis would enable the subject to take appropriate measures aiming at delaying progression or alleviate symptoms. Non-limiting examples of such measures include healthy diet and physical exercise.

As used herein, the term "prodromal" refers to the early stage of disease development where there are symptoms such as depression, sleep problems and constipation (bowel issues). Such symptoms are not PD defining. Prodromal stage symptoms precede motor symptoms, i.e. classical PD symptoms that are still not disease defining. In preferred embodiments of the invention, PD is diagnosed at the prodromal stage, or after onset of motor symptoms but before classical PD diagnosis.

In some embodiments, the present method may be used to define a subgroup of PD, i.e. to stratify a subject into a PD subgroup.

In some embodiments, biological samples may be obtained from the subject at various time points before, during, or after treatment. The level of translation in the biological sample is then determined and compared with that in a biological sample obtained from the same subject at a different time point, or with a control level obtained, for example, from a reference sample derived from an individual whose PD state is known and/or who has not been exposed to said treatment. In some embodiments, predetermined reference values obtained from a pool of apparently healthy individuals may be used as control levels in said comparisons.

Accordingly, the present invention may be used not only for diagnostic purposes but also for monitoring PD. As used herein, the term "monitoring" includes monitoring a subject's disease state or progression of PD over time, as well as monitoring any possible remission or relapse of the disease, or response to treatment. Said monitoring may be carried out by continuously assessing the level of translation and/or performing a medical test repeatedly. In some embodiments of the present invention, a subject's disease state is monitored by obtaining samples repeatedly, assaying the samples for decreased overall translation, and comparing assay results with one another and with a reference value to identify any change in the subject's disease state. Any of these aspects of the invention may be used in combination with other diagnostic tests including, but not limited to, neurological examination and brain scanning.

In some embodiments, any presently known or future preventative and/or treatment regimen may be prescribed and/or administered to subjects diagnosed with PD by the present method. Methods are thus provided for identifying a subject with PD, or increased risk of PD, and then prescribing a preventative or therapeutic regimen to said subject. Thus, some embodiments may be directed to methods of reducing risk of PD or treating PD in a subject. In some further embodiments, such methods may be carried out in the context of a clinical study.

Accordingly, the present invention may also be used for stratifying participants for clinical studies. An example here could be the choice between using Oxford Biomedica's gene therapy called ProSavin (OXB101) that is most effective in treating early stage patients or using their higher dosage drug, OXB102 which is targeted at patients in an advanced disease state.

Furthermore, the present invention may be utilized in drug screening for identifying drugs suitable for restoring normal translation in a cell. For instance, compound libraries may be screened for modulators of decreased overall translation by applying a candidate compound over a cell culture showing decreased overall translation, such as a culture of skin fibroblasts obtained from a subject with PD, and analysing the compound for any change in the level of translation by any standard technique known in the art. Normalized translation or lessened decreased overall translation by pharmacological means would be expected to prevent, cure, ameliorate or alleviate PD.

The present disclosure also provides a kit for use in diagnosing or monitoring PD in a subject. Said kit may comprise any reagents or test agents necessary for assessing protein translation as disclosed herein. Those skilled in the art can easily determine the reagents to be included depending on the specifics of the embodiment in question and a desired technique for carrying out said assessment. In some embodiments, an appropriate control sample or a threshold value may be comprised in the kit. The kit may also comprise a computer readable medium, comprising computer-executable instructions for performing any of the methods of the present disclosure.

As is apparent to a skilled person, any embodiments, details, advantages, etc. of the disclosed methods apply accordingly to other aspects of the present invention, including a kit for use in said methods, and vice versa.

It will be obvious to a person skilled in the art that, as technology advances, the inventive concept can be implemented in various ways.

### EXPERIMENTAL PART

All patient samples were obtained with appropriate permissions and informed consents in accordance with the ethical guidelines from the University of Turku and from the NINDS repository (USA).

All animal experiments were performed with appropriate permissions in accordance with guidelines for ethical conduct in the care and use of animals established by the Animal Experiment Board of Finland (Eläinkoelautakunta, permission, decision #1897).

### Example 1. LRRK2-G2019S substrates are enriched at ribosomes.

*Objective.* LRRK2 function in the brain remains largely unknown however LRRK2 has been shown to localize with mitochondria and other vesicular structures ⁸. Our preliminary findings implicated that LRRK2 function with ribosomes. We therefore set out to determine whether LRRK2-G2019S substrates were enriched at mitochondria or ribosomes. We performed an *in vitro* kinase assay using purified organelles as a source of potential substrates and LRRK2-G2019S as an active variant of LRRK2 that is relevant for PD.

*Methods.* Mitochondrial and ribosomal fractions were isolated from rat brains as previously described ^{9, 10} with modifications. 14 day old rat brains were homogenized in 12 ml homogenization buffer (300 mM sucrose, 10 mM HEPES, pH7.4, 0.1 mM EGTA, 2 mM EDTA) supplemented with protease inhibitors (1 µg/ml leupeptin, pepstatin and aprotinin and 100 µg/ml PMSF). Lysate was kept on ice for 20 min. Unbroken cells and nuclei were then removed by centrifugation at 2800 x g_{avg} for 10 min in an Eppendorf 5804R centrifuge at 4°C. Supernatant 1 (S1) was centrifuged at 22,000 x g_{avg} at 4°C for 10 min using a SW41T1 rotor and Beckman L90K ultracentrifuge. This yielded the mitochondrial pellet 2 (P2). The remaining supernatant 2 (S2) was centrifuged at 100,000 x g_{avg} at 4°C for 8 h to yield the pellet 3 (P3) which contains ribosomes and supernatant 3 (S3).

In order to compare the relative enrichment of LRRK2 substrates at ribosomes and mitochondria, we performed *in vitro* phosphorylation of fractions as follows - The P2 mitochondrial fraction and P3 ribosomal fraction from P7 rat brain was re-suspended in equal volume (300-400 µl) of *lysis buffer* (20 mM MOPS, pH 7.2, 2 mM EGTA, 2 mM EDTA, 10 % glycerol, 1 mM benzamidine) supplemented with protease inhibitors (1 µg/ml leupeptin, pepstatin and aprotinin and 100 µg/ml PMSF) and 1 % IGEPAL. After 5 min on ice, the lysate was homogenized with 8 strokes using a 27G syringe and then centrifuged at 16,000 x g at 4°C. To remove endogenous phosphates from proteins, the supernatant was supplemented with 0.1 mM ZnClz and 2.5 U Antarctic phosphatase (New England Biolabs) and incubated at 37°C for 2 h to remove covalently-bound phosphate. The phosphatase was then inactivated at 65°C for 10 min. Lysates were desalted using Micro Biospin columns from Biorad (catalogue # 732-6223). To visualize LRRK2 substrates, an *in vitro* kinase assay was carried out. The kinase reaction was started by addition of ATP (2 mM), MgClz (10 mM) and 0.9 µg/100µl (44.12 nM) of LRRK2-G2019S (Invitrogen, cat# PV4881). 5µCi of γ-[³²P]ATP was included in the reaction to monitor the efficiency of phosphate incorporation. LRKK2 reactions were incubated at 30°C for 1 h and stopped by adding *Laemmli* buffer. Reactions with radio-labelled ATP were separated by SDS-PAGE followed by Coomassie staining and phosphate incorporation was visualized by autoradiography (Figure 1 upper panels). Phosphorylation reactions that did not contain radioactive ATP were prepared in parallel. These non-radioactive samples underwent TiO2 enrichment followed by MS/MS detection (described below) to identify potential LRRK2-G2019S substrates.

*Identification of LRRK2 substrates using mass spectrometry.* 200 µg of LRRK2-G2019S phosphorylated protein (described above) was separated on 12% polyacrylamide gels and then stained, destained and sliced into 10. Samples were then reduced, alkylated and dehydrated with ACN before addition of 12.5 µg/ml MS-grade trypsin in 50mM AMBIC and the digestion was performed overnight at 37°C. Peptides were eluted, dried and dissolved in 0.1% formic acid. Samples were then incubated with TiO2 beads for 30 min in an Eppendorf Thermomixer at 800 rpm. The enriched phospho-peptides were eluted, dried and resuspended in 0.1% formic acid and separated using an Eksigent nano-LC 2D system. 8 µl of sample was loaded and washed for 15 minutes onto a pre-column (Acclaim Pep-Map 100, C18, 3 µm particle size) at a constant flow of 5 µl/min solvent A (0.1% FA in water) then separated in a 10 µm fused silica emitter, 75 µm^{∗} 16 cm Pico Tip Emitter, New Objective, packed in-house with C18 ReproSil-Pur and separated using a 78 min gradient from 3% to 90% solvent B (80%ACN) at 300 nl/min. The HPLC system was coupled to an LTQ-Orbitrap XL mass spectrometer operated in Data Dependent Acquisition mode. Spray voltage was set to 1.90 kV and the temperature of the heated capillary was set to 200 °C. The ten most intense ions from the survey scan performed by the Orbitrap were fragmented by collisioninduced dissociation in the LTQ (normalized collision energy 35, parent mass selection window 0.5 Da, 30 ms activation time and minimum signal threshold for MS/MS scans set to 100). The dynamic exclusion list was limited to a maximum of 500 masses with a maximum retention time window of 2 minutes and a relative mass window of 10 ppm. An FDR of 1% was used at the peptide level and phospho-sites were confirmed both manually and using Mascot probabilities (cutoff 90%).

*Results.* The results identified that LRRK2-G2019S kinase shows preferential activity towards proteins in ribosomal fractions whereas proteins in mitochondrial fractions were poorly phosphorylated (both fractions were normalised for total protein amount). This indicated that LRRK2-G2019S directly modifies ribosomal proteins. Using MS/MS analysis, we then identified the proteins which were phosphorylated by G2019S-LRRK2, some of which are shown (Fig. 1). These included 40S ribosomal proteins Rps6, RpS3 and RpS2 as well as eukaryotic initiation factors eIF2B5, Eif4g3 and EiF3b among others. We further validated RpS2 by *in vitro* phosphorylation of recombinant protein and found it was phosphorylated with higher specific activity by G2019S-LRRK2 than was the reported substrate M2K7 (not shown). We found that 70% of endogenous LRRK2 copurified with the P3 ribosomal fraction compared to 20% with the P2 mitochondrial fraction (not shown).

*Conclusions.* Together these findings highlighted a prominent association of LRRK2 with ribosomes and identified several potential substrates for LRRK2-G2019S there that could be detected using phospho-antibodies or SRM assays as a test of PD.

### Example 2. Protein translation is impaired in neurons following exposure to rotenone and LRRK2 activity is increased.

Understanding the molecular mechanism underlying PD requires the use of cell and animal models that reliably replicate the disease hallmarks. Rotenone is one such model. It is a naturally occurring insecticide that evokes the anatomical and behavioural features of PD in rodents ^{4, 5}, while in humans, rotenone is one of the environmental factors associated with increased risk to develop PD 6.

*Methods.* Measurement of protein translation using various S35-methioine-labelling - In order to model PD in vitro, hippocampal neurons were metabolically labelled with S35-methionine. Hippocampal neurons in 24 well plates were transferred to Met/Cys-free medium (Invitrogen, Gibco). Cells were treated with for 30 min 37C, 5% CO2. 500µl per well of labeling medium (100µCi Met-S35/ml) was added and cells grown at 37°, 5% CO2 for 2hr in the presence or absence of rotenone (0.01-10 µM). Cells were washed and lysed in 75µl Laemmli lysis buffer. Samples were separated by SDS PAGE, incubated with EN3HANCE (NEN), gels dried and exposed to auto-radiographic film with intensifying screen to determine relative levels of de novo protein synthesis.

*Results.* Treatment of neurons with rotenone dose-dependently decreased de novo protein synthesis as determined using S35-methioine labeling (Figure 2).

*Conclusions.* Translation of mRNA may be impaired in PD and may contribute to the underlying pathology.

### Example 3. Rotenone activates LRRK2.

*Objective.* We wanted to test whether LRRK2 was activated in the rotenone model which replicates the neuropathological features of PD, inducing highly specific degeneration of nigral-striatal dopaminergic neurons, Lewy body formation and L-DOPA behavioural deficits in rats ^{5, 7}. We also wanted to determine the effect of LRRK2-G2019S on protein translation.

*Methods.* To examine the effect of rotenone treatment on LRRK2 activity, BHK21 cells were treated with or without the indicated dose of rotenone for 4h. Cells were lysed in *Laemmli buffer* and proteins separated by SDS PAGE. LRRK2 activity was determined by immunoblotting with an antibody detecting phospho-S935-LRRK2. Blots were reprobed with an antibody detecting LRRK2 to establish loading variability. LRRK2 activity was quantified by densitometry of gel exposures captured using the Gel Doc XR system (Biorad).

To determine whether inhibition of LRRK2 activity could recover normal translation in BHK21 cells following rotenone treatment, BHK21 cells with or without prior treatment (30 min) with LRRK2 inhibitor (1 or 10 µM of IN1) were treated with rotenone or carrier (control) as indicated. Translation was visualized following AHA labeling and immunofluorescence quantified as earlier.

To determine whether LRRK2-G2019S directly impacted protein translation, we utilized a cell-free protein expression system from Takara Bio Inc. (Japan). A β-galactosidase reporter plasmid (150 ng) was incubated with 60 nM of GST-G2019S-LRRK2 or GST alone in a 5 µl reaction for 1.5 h at 32°C. The amount of β-galactosidase was estimated using colorimetric detection, from duplicate samples using a 96-well format. For every 2 µl of reaction mix, 100 µl of ortho-Nitrophenyl-β-galactoside (ONPG) buffer (ONPG 0.66 mg/ml, 1mM MgCl2, 45 mM β-mercaptoethanol, 0.1 M sodium phosphate buffer, pH 7.2) was added. The reaction was stopped by adding *stop solution* (100 mM glycine, NaOH, pH 12.8) 100 µl/well after 15 min. Absorbance at 420 nm was measured with the Synergy H1 Hybrid Reader (Biotek).

*Results.* Rotenone dose dependently increased LRRK2 activity in intact cells (Figure 3).

### Example 4. Lrrk2 inhibition protects dopaminergic neurons from rotenone-induced atrophy.

*Objective.* To establish whether Lrrk2 activation in response to rotenone induced neurite retraction, a hallmark of Parkinson's Disease, we measured the extent of neurite atrophy in cultured dopaminergic neurons exposed to rotenone.

*Methods.* Rotenone treatment of cell cultures - Midbrain neurons from P0 rat were plated at a density of 200,000 cells per well. At 3 days in vitro, cells were treated with rotenone in DMSO with or without IN1, GSK-2578215A of MLi-2 at the concentrations indicated. After 24 h, cells were fixed with 4 % PFA in PBS and tyrosine hydroxylase (TH) was detected by standard immunostaining methods obvious to those skilled in the art. Cells were imaged using a Zeiss AxioVert 200M microscope. To measure atrophy, the % of TH-positive neurons with intact neurites was scored.

*Results.* Rotenone induced atrophy of dopaminergic neurons. This was prevented by treatment with all three Lrrk2 inhibitors. Inhibition of Lrrk2 alone, in the absence of rotenone, did not significantly alter neurite length.

*Conclusions.* Lrrk2 activity promotes dopaminergic neuron atrophy, a hallmark of Parkinson's pathology.

### Example 5. Protein translation is increased in hippocampal neurons upon inhibition of LRRK2 with structurally independent molecules.

*Objective.* To test whether Lrrk2 regulated ribosomal function in neurons, we measured the effect of three structurally independent inhibitors of Lrrk2 on *de novo* protein synthesis using non-canonical amino acid labeling. Rat hippocampal neurons were labelled using L-azido-homoalanine (AHA), a methionine analogue, followed by copper-I-catalyzed alkyne-azide cycloaddition of Alexa-488 with an alkyne moiety to visualize newly synthesized proteins, as previously described ⁸. The inhibitors used were LRRK2-IN1, which blocks Lrrk2 with a biochemical IC50 of 0.01 µM ⁹, GSK-2578215A, a highly specific and potent LRRK2 inhibitor ¹⁰ and 3-(4-Pyrinimidyl) Indazole (MLI-2), a selective orally active inhibitor of LRRK2 ¹¹.

*Methods.* Measuring protein translation in hippocampal neurons using FUNCAT. To measure protein translation, hippocampal neurons were plated at densities of 100,000 cells per well on round coverslips (1.3 cm diameter) in 24-well plates. Protein translation was measured following 20h after plating. Cells were washed once and incubated with methionine-free DMEM medium (Gibco, Thermo Fisher Scientific) supplemented with 2 mM glutamine. Then 1 mM L-azidohomoalanine (AHA) was added for 30 min. Cells were washed with 1 ml PBS and fixed with 4 % paraformaldehyde. LRRK2 inhibitors, when used, were added in the methionine-free medium and retained throughout the AHA labelling procedure. Cells were permeabilized in block solution (0.25 % Triton X100, 0.2 % BSA, 5 % sucrose, 10 % horse serum in PBS), and washed 5 times with 1 ml of 3 % BSA in PBS. In situ labeling with AHA was achieved using a click-it reaction to covalently Alexa-488, using reagents from ThermoFisher Life Technologies (catalogue # A10267). Coverslips were mounted in Mowiol containing 2.5 % 1,4-Diazabicyclo[2.2.2]octane. Fluorescent images were acquired using a Leica DMRE microscope and Hamamatsu Photonics ORCA C4742-95 CCD camera and Zen software (Carl Zeiss). Mean fluorescence intensities were measured from regions of interest in the perisomal region. Dendrite measurements were taken from line intensities (10 µm lines from the soma into the dendrite).

*Results.* Lrrk2 inhibitors (GSK-2578215, LRRK2-IN1 and MLi-2) significantly increased protein synthesis as measured in hippocampal neuron somas (figure 5A a-b, 5B a, b) and dendrites (figure 5A d-e, 5B a, c) of treated hippocampal neurons at doses as low as 0.1 µM (GSK-2578215 and LRRK2-IN1) and 0.01µM (MLi-2) respectively. This indicates that basal Lrrk2 activity has a repressive effect on protein synthesis in neurons. As a negative control, we treated neurons with anisomycin, a ribotoxic inhibitor of peptidyl transferase. This treatment effectively inhibited de novo protein synthesis in this assay (figure 5B c, f).

*Conclusions.* LRRK2 represses mRNA translation into nascent proteins in hippocampal neurons.

### Example 6. Protein translation is increased in dopaminergic neurons upon inhibition of LRRK2 with structurally independent molecules.

*Objective.* To determine if LRRK2 inhibition alters protein translation in midbrain cultures and more specifically, in dopaminergic neurons.

*Methods.* To measure protein translation in dopaminergic neurons, mid brain cultures were plated at densities of 500,000 cells per well on round coverslips (1.3 cm diameter) in 24-well plates. At 3 days post-plating neurons underwent FUNCAT (see example 4). Dopaminergic neurons were detected by tyrosine hydroxylase immunofluorescence. Fluorescent intensity was measured as described in example 4 (methods).

*Results.* Inhibition of LRRK2 using two structurally independent inhibitors, increased protein translation in dopaminergic neurons (Figure 5g-j, figure6a-c). Anisomycin (an inhibitor of translation) was added as a negative control. It effectively reduced translation as determined using fluorescence (figure 5k, l, figure 6a-c).

*Conclusions.* These findings indicate that constitutive LRRK2 activity represses protein translation in dopaminergic neurons and is consistent with the idea that the gain of function *LRRK2-G2019S* mutant may be relevant for the neuropathology of PD.

### Example 7. Measuring nascent protein synthesis in patient skin fibroblasts provides a functional readout for PD.

*Objective.* Since Lrrk2 is a ubiquitously expressed protein, we wanted to test whether peripheral cells from PD patients (obtained from a skin punch) would display impaired translation. Fibroblasts from control individuals and clinically diagnosed patients with sporadic or G2019S PD were obtained from the National Institute of Neurological Disorders and Stroke (NINDS-Coriell) Repository.

*Methods.* For figure 7, we measured mRNA translation in fibroblasts cultivated from patient skin biopsies using the non-canonical labelling approach outlined in example 4 with the following modifications. Fibroblasts were passaged upon reaching 95% confluence and assayed at passage 7-9. For measurement of nascent protein synthesis, fibroblasts at 48 h post plating were depleted of methionine and incubated with AHA, followed by AHA-click conjugation of Alexa-488 as described in the methods section of example 4. Average somatic fluorescent intensities were measured. As not all fibroblast lines could be assayed simultaneously, patient lines were paired with one or more control lines of equivalent passage and assayed on the same day. For figure 8, we tested whether the reduced protein synthesis observed in patient cells could be reversed upon treatment with 1 µM LRRK2 inhibitor-1 (IN1) or 0.01µM MLi-2.

*Results.* In skin fibroblasts from LRRK2-G2019S carriers and sporadic cases, translation was repressed (figure 7). This repression of protein synthesis was reversed upon inhibition of LRRK2 activity with structurally independent-LRRK2 inhibitors IN1 or MLi-2 (figure 8).

*Conclusion.* These results indicate that LRRK2 suppresses protein synthesis in peripheral cells from G2019S carriers and sporadic PD patients alike. This finding is consistent with the PD-associated EIF4G1 mutations that negatively regulate mRNA translation ^{11.} These data indicate that LRRK2 repression of ribosome function provides a convergent mechanism that is present in familial and sporadic PD.

### Example 8. Protein synthesis is reduced in probable early stage PD patients.

*Objective.* To test if "decreased overall translation" could be observed at an early stage of PD.

*Methods.* We collected skin punches from the upper arm of *probable* Parkinson's patients at Turku University Hospital (T.U.H). Skin punches from agematched controls (spouse or sibling) were collected at the same time. Most of these patients had not been diagnosed with [123I]-FP-CIT-SPECT imaging for DAT binding defect at the time of biopsy. Skin punches were placed directly in minimal essential medium (MEM, Sigma Aldrich) and dissected within 1 hour of extraction. The fatty tissue from the hypodermal layer was removed and remaining dermal and epidermal layers were chopped into small 2mm pieces that were then maintained in MEM supplemented with glutamine (2 mM), penicillin (50 U/ml) and streptomycin (50 µg/ml) at 37C and 5% CO2. Fibroblast cells grew out from these explants within days of plating.

*Results.* Fibroblast cells derived from patients in the early stage of PD (ages 52 - 69 years) compared to the NINDs cohort (example 6), showed significantly reduced protein synthesis compared to control individuals (Figure 9). As before, mRNA translation was measured using AHA-labeling and fluorence detection (example 7). Combining T.U.H. and NINDS cohorts yielded a size effect of 1.86. The receiver operating characteristic curve (ROC) analysis for the combined cohorts gave an area under the curve value of 93% indicating that this measurement provides good predictive power (Figure 10a). Protein synthesis correlated negatively with age in patients (figure 10b), but not in healthy individuals (Figure 10c). This suggests that translational inhibition is a disease-specific feature that progresses with age and is not associated to normal ageing in healthy individuals. *Conclusions.* Measurement of nascent protein synthesis can provide a biomarker readout of both sporadic and G2019S PD in the early stages.

### Example 9. Protein synthesis from patient fibroblasts can be measured in a multi-well format.

*Objective.* To determine if the individual cell fluorescence measurements (figure 7-9) could be simplified in a multi-well format using a spectrophotometric readout that is amenable to scale up (figure 11).

*Methods.* Fibroblasts from patients and control individuals were isolated as described for example 7. For measurement of protein translation in multi-well format, cells were plated at a density of 30,000 per 48-well plate well in DMEM. Cells were incubated in methionine-free DMEM for 30 min essentially as described in the methods for example 4, and 1mM AHA was added. After 30 min, cells were fixed with 4% paraformaldehyde containing 4% sucrose for 30 min at RT. Cells were permeabilized overnight in blocking buffer (0.25% TX100, 0.2% BSA, 5% sucrose, 10% horse serum in PBS). Cells were washed 3x with PBS-BSA (3%). The click-it reaction was carried out as described in example 4 to link AHA with Alexa-488 using the kit from ThermoFisher Life Technologies (catalogue # A10267), although any standard click-it reaction protocol should work. The reaction was carried out for 30 min. Cells were then washed 5x with PBS-BSA and maintained at 4°C in PBS. Fluorescence was detected using a BioTek Synergy H1 multi-mode plate reader using 350/461 nm filters. Background fluorescence was determined from wells with cells that did not undergo AHA labeling and subtracted.

*Results.* The results show that protein translation is inhibited in fibroblasts from PD patients (figure 11).

*Conclusions.* This example shows that it is possible to measure nascent protein synthesis in a simple multi-well, fluorescence-detection format and that this yields a quantitative biomarker for PD.

### Example 10. Measurement of translation in patient fibroblasts and peripheral blood mononuclear cells (PBMCs) using AHA-labelling provides a massspectrometry (MS)-based method to detect impaired translation.

*Objective.* To test whether MS-based protein sequencing could be applied to identify de novo synthesized proteins in patient and healthy samples from skin or blood.

*Methods. Isolation of peripheral blood mononuclear cells (PBMCs).* Blood (7-9ml) was collected into vacutainer tubes (BD) containing heparin and PBMCs were isolated within 1 hr of extraction from patients or volunteers as follows: blood was diluted 1:1 with sterile PBS and 6 ml was layered on 4,5 ml Ficoll Paque Plus solution (Amersham Biosciences, #17-1440-002) in 15 ml Falcon tubes. Tubes were centrifuged at 400 RCF for 30 min at RT. The PBMC cell layer was collected into sterile PBS and washed twice in 30 ml of sterile PBS. The cell pellet was resuspended in methionine-free RPMI-1640.

*AHA labeling of PBMCs.* To label PBMCs with L-azido-hydroalanine (AHA), cells were plated in dishes in 1ml of methionine-free RPMI-1640 as above and incubated for 30min at 37C, 5% CO2. Medium was replaced with RPMI containing 2 mM AHA (Baseclick) and incubated. Cells were then pelleted and 100µl of lysis buffer (10 mM HEPES, pH 7.4, 2 mM EGTA, 50 mM b-glycerophosphate, 1mM DTT, 1 mM Na3VO4, 1% TX100, 10 % glycerol, 1 % SDS, 40 mM NaF, 1 mM benzamadine. Protease inhibitors (1µg/ml), leupeptin, pepstatin and 100 µg/ml PMSF were added fresh before use). 8M urea and 250 U/ml of benzonase (MERCK #70746) were also added. Cells were incubated for 15 min on ice and centrifuged at 13,400 RCF for 5 min at 4°C to remove unbroken cells. Supernatants underwent a cyclo-addition reaction to attach AHA-labelled proteins to alkyne agarose using the Invitrogen kit (#C10269), though any click chemistry protocol should work. Components A, C and B were mixed at a ratio of 5:5:1 (click reaction). Alkyne agarose (25µl per sample) was washed with milli-Q. 50µl of click reaction mix was added to the beads followed by addition of 400 ug of lysate. The mix was vortexed and incubated at RT with gentle rotation. Beads were then washed with milli-Q followed by SDS wash buffer. 1ml of SDS wash buffer, pH 8.5, containing 7mM iodoacetamide was added and the sample was incubated in the dark for 30 min. The agarose was washed in 1 ml SDS wash buffer pH 8.5 containing 5mM DTT and stored at 4°C before preparing for trypsinization and mass spectrometry analysis. Resin bound proteins were digested overnight with Trypsin, and collected peptides were cleaned with C18 UltraMicroSpin columns and analyzed by MS/MS.

*AHA labelling of fibroblasts.* Patient fibroblasts (see example 7), were plated at 100 k per 6 cm dish in 5 ml DMEM supplemented with 10% FBS, 2 mM Gln, penicillin/streptomycin and incubated at 37C in 5% CO2. Before AHA labelling, medium was replaced with methionine-free, serum-free RPMI-1640 and incubated for 30 min. Medium was then replaced with 3 ml of serum-free/methionine-free RPMI containing 2 mM AHA (Baseclick) and incubated for 14h. Cells were collected by scraping gently and centrifuging at 400 RCF for 5 min at 4°C. Lysates were prepared as above for PBMCs using 100 µl lysis buffer per sample. Samples that were treated with Methionine instead of AHA served as negative controls. Binding of AHA-labelled proteins to beads, trypsinization and MS/MS analysis was done as described above for PBMC samples. The resulting RAW data files were analysed and quantified in MaxQuant with 0.05% FDR rate. After removal of reverse sequences and sequences that did not reach a threshold score, peptides were merged to provide protein intensities. These represent the amount of a given protein in the sample.

*Results.* The results show that protein translation can be measured in non-invasively obtained PBMCs and in fibroblasts obtained from patient skin biopsies by enrichment of AHA-labeled proteins followed by mass spectrometry detection of trypsinized proteins. This identifies *de novo* protein synthesis signatures that can be used to define patient status (figure 12a). Also, the detection of conjugated AHA groups specifically in AHA-labelled samples validates that the method enriches for AHA-labelled proteins (figure 12b).

*Conclusions.* This provides a method whereby a functional readout of protein translation as well as protein and/or peptide signatures can be obtained from patient samples using mass spectrometry detection.

### Example 11. Protein synthesis can be measured using AHA-labelling followed by alkyne-biotin-neutravidin enrichment and antibody (or mass spectrometry) detection in patient cells.

*Objective.* To determine if antibody detection can be used to monitor levels of translation in patient cells.

*Methods.* PBMCs were isolated as described for example 10. Fibroblasts were isolated and cultivated as described for examples 7 and 10. For both cell types, methionine starvation and AHA labelling was carried out as described for example 10. Cells were lysed in 100µl of hypotonic lysis buffer (10 mM HEPES, pH 7.4, 2 mM EGTA, 50 mM beta-glycerophosphate, 1m M DTT, 1 mM Na3VO4, 1% TX100, 10 % glycerol, 40 mM NaF, 1 mM benzamidine containing 1µg/ml leupeptin and pepstatin and 100 µg/ml PMSF, added fresh before use). Component A (10 µl), component B (2 µl) and component C (10µl) were mixed. 0.2µl of Biotin-alkyne (Lumiprobe: C37B0) was added as indicated. Lysate was mixed 50:50 with beads and incubated for 20h at RT with gentle mixing. For reactions with alkyne-biotin, reaction tubes were gently vortexed and incubated for 30 min at RT. Excess reagents (including free biotin) were removed by gel filtration using a PD-spintrap G25 column (5kDa cut-off) (Amersham Pharmacia) and hypotonic lysis buffer. Biotin-AHA-tagged proteins were immobilized on 20µl (bed volume) neutravidin affinity sepharose resin (Pierce) by incubating for 30 min at RT.

Sepharose was washed 2x with 1 ml LiCl buffer (500 mM LiCl2, 100 mM Tris, pH 7.6, 0.1 % TX100, 1mM DTT), followed by 4 x 1ml PBS washes. Samples were then lysed in Laemmli, boiled for 10 min and separated by SDS PAGE. Samples were either in-gel digested with trypsin for MS/MS identification (example 1) or transferred to nitrocellulose for quantitative immunoblot detection using an anti-biotin, horse-radish peroxidase (HRP)-conjugated antibody (Cell Signalling Technology) (example 9b). The relative amounts of AHA-tagged proteins were visualized from chemilumenescence detections and scanned with a Molecular Imaging Gel-doc system (Biorad). Anisomycin, an inhibitor of protein translation, was added as a negative control.

*Results.* AHA labelling of PBMCs or fibroblasts from patients enables sensitive measurement of protein synthesis using antibody detection. AHA labelling of PBMCs or fibroblasts from patient cells facilitates enrichment of nascent proteins for MS/MS detection following in gel digestion (Figure 12c).

*Conclusions.* This provides a method whereby a functional readout of protein translation can be obtained from control and patient blood or skin samples using enrichment followed by antibody/enzymatic detection.

### Example 12. Mass spectrometry identifies impaired levels of translation in cells from sporadic and G2019S PD cases compared to healthy controls.

*Objective.* To determine if significant differences between translation in control and PD patient cells can be detected using mass spectrometry.

*Methods.* For figure 13, sample preparation was essentially as described for example 10. Corresponding negative controls were included for each sample. These consisted of cells that were not labelled with AHA, but with methionine. The LTQ-Orbitrap XL settings were as described in example 1. The resulting RAW data files were analysed and quantified in MaxQuant with 0.05% FDR rate. Quantified protein results from MaxQuant were imported into Perseus software, where false positive reverse identifications were filtered away. Raw intensity values were log2 transformed, missing values imputed by 1 and proteins were clustered with Hierarchial clustering in Perseus with Euclidian distance and average linkage. Samples were combined from three independent cohorts (NINDS-Coriell, T.U.H. and European Biobank) as shown in Table 1.

*Results.* Clear protein intensity differences were visible between patient groups sporadic and G2019S verses control, merely by visualising heatmaps of protein intensity (figure 13). Significantly impaired translation was found when comparing sporadic PD patient de novo synthesized protein intensities to healthy controls. 39 significantly altered proteins were identified (figure 14). Significantly impaired translation was found when comparing G2019S PD patient *de novo* synthesized protein intensities to healthy controls. 50 significantly altered proteins were identified (figure 15).

*Conclusions.* We identified separate panels of proteins that are significantly altered in sporadic and G2019S PD patients compared to healthy controls.

### Example 13. Signatures predicting patient status: Normal Healthy verses sporadic PD.

*Objective.* To identify single biomarkers or biomarker signatures resulting from impaired translation that will help distinguish sporadic PD from normal (healthy) individuals.

*Methods.* Lists of protein intensities that were significantly altered in sporadic PD verses controls were trained using the Glmnet package to fit a linear regression model and to identify which features give the best prediction of disease status. For all predictions, we used all patient data from three cohorts as earlier (NINDS-Coriell, TUH, EB). In the first figure, we used the list of significantly changing proteins from sporadic verses control healthy individuals using no additional thresholding. This yielded a signature of 32 features that predicted patient status (Sporadic PD or Healthy) with a prediction score of 93% (figure 16). We next imposed a cut off of two fold change. We included only proteins where levels were significantly changed more than two-fold compared to controls. This yielded a signature with 14 features and 99% prediction score using Glmnet parameters alpha=0.01, lamba = 0.005 (figure 17) using Glmnet parameters alpha=0.1, lambda= 0.005. We next used *brut force* to identify which pair of features from this signature gave the best prediction score. This yielded SNX2 and LIMA1 as a dual feature signature with a prediction score of 88% using Glmnet parameters alpha=1, lambda= 0.056 (figure 18).

*Results.* We identified three signatures comprising 32, 14 or 2 features respectively with prediction accuracy of 93%, 99% and 88%.

*Conclusions.* These signatures should be used to help predict PD in non-inherited forms of PD. They can be used to screen patient samples, skin fibroblasts, blood or other bodily fluid such as cerebral spinal fluid or urine.

### Example 14. Signatures predicting patient status: Normal Healthy verses G2019S PD.

*Objective.* To identify single biomarkers or biomarker signatures resulting from impaired translation that will help distinguish G2019S PD from normal (healthy) individuals.

*Methods.* Lists of protein intensities that were significantly altered by more than two-fold in G2019S PD patients compared to control individuals were trained using the Glmnet package to a fit linear regression model and identify which features give the best prediction of disease status, as in example 13. For all predictions, we used all patient data from three cohorts as earlier (NINDS-Coriell, TUH, EB). This yielded a signature with 13 features and prediction accuracy score of 98% using Glmnet parameters alpha=0.1, lambda= 0.003 (figure 19). We then used brut force to identify a single pair of makers with highest prediction score for this subgroup of patients. This yielded FAM120A and ADAR as two biomarkers that when tested for together give a prediction score of 98% (figure 20).

*Results.* We identified two signatures derived from 13 or 2 features respectively, with prediction accuracy of 98%.

*Conclusions.* These signatures should be used to help predict PD in inherited forms of PD. They can be used to screen patient samples, skin fibroblasts, blood or other bodily fluid such as cerebral spinal fluid or urine.

### References

1. Nalls, M.A., et al. Large-scale meta-analysis of genome-wide association data identifies six new risk loci for Parkinson's disease. Nat Genet 46, 989-993 (2014).
2. Haugarvoll, K. & Wszolek, Z.K. Clinical features of LRRK2 parkinsonism. Parkinsonism Relat Disord 15 Suppl 3, S205-208 (2009).
3. WO 2015257514 A1 , RIBOSOMAL PROTEIN S15 PHOSPHORYLATION MEDIATES LRRK2 NEURODEGENERATION IN PARKINSON'S DISEASE.
4. Poewe, W., et al. Parkinson disease. Nat Rev Dis Primers 3, 17013 (2017).
5. The "Cell Line and DNA Biobank from Patients Affected by Genetic Diseases", member of the Telethon Network of Genetic Biobanks (project no. GTB12001), funded by Telethon Italy, provided us with specimens.
6. Ota, K.T., et al. REDD1 is essential for stress-induced synaptic loss and depressive behavior. Nat Med 20, 531-535 (2014).
7. Kay, D.M., et al. Validity and utility of a LRRK2 G2019S mutation test for the diagnosis of Parkinson's disease. Genet Test 10, 221-227 (2006).
8. Biskup, S., et al. Localization of LRRK2 to membranous and vesicular structures in mammalian brain. Ann Neurol 60, 557-569 (2006).
9. Arnstein, H.R., Cox, R.A., Gould, H. & Potter, H. A comparison of methods for the isolation and fractionation of reticulocyte ribosomes. Biochem J 96, 500-506 (1965).
10. Zhao, Y., et al. The JNK inhibitor D-JNKI-1 blocks apoptotic JNK signaling in brain mitochondria. Mol Cell Neurosci 49, 300-310 (2012).
11. Scott, J.D., et al. Discovery of a 3-(4-Pyrimidinyl) Indazole (MLi-2), an Orally Available and Selective Leucine-Rich Repeat Kinase 2 (LRRK2) Inhibitor that Reduces Brain Kinase Activity. J Med Chem 60, 2983-2992 (2017).

## Claims

1. A method of diagnosing Parkinson's disease (PD) in a subject, said method comprising testing a sample obtained from the subject for changes in the overall level of translation of mRNA into de novo synthesised proteins, wherein decreased overall level of translation of mRNA into de novo synthesised protein as compared to that of a healthy control is indicative of PD.

2. A method of monitoring PD in a subject, said method comprising testing a sample obtained from the subject at different time points for changes in the overall level of translation of mRNA into de novo synthesised proteins, wherein decreased overall level of translation of mRNA into de novo synthesised proteins over time is indicative of progression of PD.

3. The method according to claim 1 or 2, wherein a protein expression signature comprising decreased expression of SNX2 is indicative of decreased overall level of translation of mRNA.

4. The method according to claim 3, wherein the protein expression signature indicative of decreased overall level of translation of mRNA further comprises decreased expression of LIMA1.

5. The method according to claim 4, wherein the protein expression signature indicative of decreased overall level of translation of mRNA further comprises RRS1, TFRC, RASA1, NRAS, KRT36, Q56UQ5, PPIC, TRIM25, JAGN1, WDR36, CSE1L, and RPL27.

6. The method according to claim 5, wherein the protein expression signature indicative of decreased overall level of translation of mRNA further comprises MX1, APOL2, DBI, ADA1B0GVG8, ERLEC1, TXNL1, TMSB10, CCDC50, BAG3, RASA1, CHMP4B, EIF2S2, HNRNPH1, NACAGNL3, PSMC1, ACOX1, TMEM167A and ASNS.

7. The method according to claim 1 or 2, wherein a protein expression signature indicative of decreased overall level of translation of mRNA comprises one or more proteins encoded by genes selected from the group consisting of SNX2, LIMA1, RRS1, TFRC, RASA1, NRAS, KRT36, Q56UQ5, PPIC, TRIM25, JAGN1, WDR36, CSE1L, RPL27, MX1, APOL2, DBI, ADA1B0GVG8, ERLEC1, TXNL1, TMSB10, CCDC50, BAG3, RA-SA1, CHMP4B, EIF2S2, HNRNPH1, NACAGNL3, PSMC1, ACOX1, TMEM167A and ASNS.

8. The method according to any one of claims 1 to 7, wherein said PD is sporadic PD.

9. The method according to claim 1 or 2, wherein a protein expression signature comprising decreased expression of FAM120A and ADAR is indicative of decreased overall level of translation of mRNA.

10. The method according to claim 9, wherein the protein expression signature indicative of decreased overall level of translation of mRNA further comprises MAP1A, COL5A, BCL9L, USP7, RECK, CNOT7, NKIRAS1, ARPC5, CPN31, TMEM208 and CRIP2.

11. The method according to claim 10, wherein the protein expression signature indicative of decreased overall level of translation of mRNA further comprises GDI1, USP9X, ADRM1, PEF1, YIF1B, CANX, GHITM, CANX, TNS1, OGDH, TNPO1 PEPD, HNRNFA3, MAP4, ALDH18A1, DDX21, DERL1, TPM2, NPM1, CLIC4, HLA-A, CDC41, STT3A, RHOA, CTNNB1, TRAM1, PLS3, SSR3, CTSK, NCBP1, PTRH2, SLC35B2, SF3B3, SLC2A14, SLC2A3, NUCB2, HEL-S-109, SEC61G, CLPTM1, RECK, PRMT1, ABCF2, VAMP7, RTCA, AEBP1, CPXM2, S100A16, RRAGD, RRAGC, EHD1 and PALLD.

12. The method according to claim 1 or 2, wherein a protein expression signature indicative of decreased overall level of translation of mRNA comprises one or more proteins encoded by genes selected from the group consisting of FAM120A, ADAR, MAP1A, COL5A, BCL9L, USP7, RECK, CNOT7, NKIRAS1, ARPC5, CPN31, TMEM208, CRIP2, GDI1, USP9X, ADRM1, PEF1, YIF1B, CANX, GHITM, CANX, TNS1, OGDH, TNPO1 PEPD, HNRNFA3, MAP4, ALDH18A1, DDX21, DERL1, TPM2, NPM1, CLIC4, HLA-A, CDC41, STT3A, RHOA, CTNNB1, TRAM1, PLS3, SSR3, CTSK, NCBP1, PTRH2, SLC35B2, SF3B3, SLC2A14, SLC2A3, NUCB2, HEL-S-109, SEC61G, CLPTM1, RECK, PRMT1, ABCF2, VAMP7, RTCA, AEBP1, CPXM2, S100A16, RRAGD, RRAGC, EHD1 and PALLD.

13. The method according to any one of claims 9 to 12, wherein said subject is a carrier of G2019S or belongs to a subgroup of sporadic PD patients.

14. The method according to any one of claims 1 to 13, wherein the overall level of translation of mRNA is assessed by a method selected from the group consisting of mass spectrometry including selected reaction monitoring (SRM), multiple reaction monitoring (MRM), parallel reaction monitoring (PRM), data dependent acquisition (DDA) or data independent acquisition (DIA), metabolic labelling and click chemistry, or where the agent is an antibody.

15. The method according to any one of claims 1 to 14, wherein the protein expression signature is used in a non-comparative or comparative manner.

16. The method according to any one of claims 1 to 15, wherein changes in post-translational modifications assessed either quantitatively or qualitatively by mass spectrometry or antibody, are indicative of changes in the overall level of translation of mRNA.

17. The method according to claim 16, wherein changes in structure of the post-translational modifications are indicative of decreased overall level of translation of mRNA.

18. The method according to any one of claims 2 to 16, wherein said monitoring comprises monitoring progression of PD, monitoring response to treatment, monitoring remission of PD, or monitoring relapse of PD.

19. The method according to any one of claims 1 to 17, wherein said diagnosing comprises stratifying said subject into a subgroup of PD.

20. The method according to any preceding claim, wherein said sample is a skin biopsy sample, a blood sample, a cell sample, a tissue sample, or a bodily fluid.

21. Use of a kit for diagnosing, staging or monitoring PD, or stratifying PD patients based on changes in SNX2, FAM120A or ADAR expression, wherein said kit comprises one or more reagents for testing a sample for changes in SNX2, FAM120A or ADAR expression.

## Patentansprüche

1. Verfahren zur Diagnose der Parkinson-Krankheit (PD) bei einer Versuchsperson, wobei das Verfahren das Testen einer von der Versuchsperson erhaltenen Probe auf Änderungen beim Gesamtniveau der Translation von mRNA in de novo synthetisierte Proteine umfasst, wobei das verminderte Gesamtniveau der Translation von mRNA in de novo synthetisiertes Protein im Vergleich zu demjenigen einer gesunden Kontrollperson auf die PD hinweist.

2. Verfahren zum Überwachen der PD bei einer Versuchsperson, wobei das Verfahren das Testen einer von der Versuchsperson an verschiedenen Zeitpunkten erhaltenen Probe auf Änderungen beim Gesamtniveau der Translation von mRNA in de novo synthetisierte Proteine umfasst, wobei das verminderte Gesamtniveau der Translation von mRNA in de novo synthetisierte Proteine über die Zeit auf ein Fortschreiten der PD hinweist.

3. Verfahren nach Anspruch 1 oder 2, wobei eine Proteinexpressionssignatur, die verminderte Expression von SNX2 umfasst, auf ein vermindertes Gesamtniveau der Translation von mRNA hinweist.

4. Verfahren nach Anspruch 3, wobei die Proteinexpressionssignatur, die auf ein vermindertes Gesamtniveau der Translation von mRNA hinweist, ferner eine verminderte Expression von LIMA1 umfasst.

5. Verfahren nach Anspruch 4, wobei die Proteinexpressionssignatur, die auf ein vermindertes Gesamtniveau der Translation von mRNA hinweist, ferner RRS1, TFRC, RASA1, NRAS, KRT36, Q56UQ5, PPIC, TRIM25, JAGN1, WDR36, CSELL und RPL27 umfasst.

6. Verfahren nach Anspruch 5, wobei die Proteinexpressionssignatur, die auf ein vermindertes Gesamtniveau der Translation von mRNA hinweist, ferner MX1, APOL2, DBI, ADA1B0GVG8, ERLEC1, TXNL1, TMSB10, CCDC50, BAG3, RASA1, CHMP4B, EIF2S2, HNRNPH1, NACAGNL3, PSMC1, ACOX1, TMEM167A und ASNS umfasst.

7. Verfahren nach Anspruch 1 oder 2, wobei eine Proteinexpressionssignatur, die auf ein vermindertes Gesamtniveau der Translation von mRNA hinweist, eines oder mehrere Proteine umfasst, die durch Gene codiert werden, die ausgewählt werden aus der Gruppe, die besteht aus SNX2, LIMA1, RRS1, TFRC, RASA1, NRAS, KRT36, Q56UQ5, PPIC, TRIM25, JAGN1, WDR36, CSELL, RPL27, MX1, APOL2, DBI, ADA1B0GVG8, ERLEC1, TXNL1, TSMB10, CCDC50, BAG3, RA-SA1, CHMP4B, EIF2S2, HNRNPH1, NACAGNL3, PSMC1, ACOX1, TMEM167A und ASNS.

8. Verfahren nach einem der Ansprüche 1 bis 7, wobei die PD sporadische PD ist.

9. Verfahren nach Anspruch 1 oder 2, wobei eine Proteinexpressionssignatur, die verminderte Expression von FAM120A und ADAR umfasst, auf ein vermindertes Gesamtniveau der Translation von mRNA hinweist.

10. Verfahren nach Anspruch 9, wobei die Proteinexpressionssignatur, die auf ein vermindertes Gesamtniveau der Translation von mRNA hinweist, ferner MAP1A, COL5A, BCL9L, USP7, RECK, CNOT7, NKIRAS1, ARPC5, CPN31, TMEM208 und CRIP2 umfasst.

11. Verfahren nach Anspruch 10, wobei die Proteinexpressionssignatur, die auf ein vermindertes Gesamtniveau der Translation von mRNA hinweist, ferner GDI1, USP9X, ADRM1, PEF1, YIF1B, CANX, GHITM, CANX, TNS1, OGDH, TNPO1 PEPD, HNRNFA3, MAP4, ALDH18A1, DDX21, DERL1, TPM2, NPM1, CLIC4, HLA-A, CDC41, STT3A, RHOA, CTNNB1, TRAM1, PLS3, SSR3, CTSK, NCBP1, PTRH2, SLC35B2, SF3B3, SLC2A14, SLC2A3, NUCB2, HEL-S-109, SEC61G, CLPTM1, RECK, PRMT1, ABCF2, VAMP7, RTCA, AEBP1, CPXM2, S100A16, RRAGD, RRAGC, EHD1 und PALLD umfasst.

12. Verfahren nach Anspruch 1 oder 2, wobei eine Proteinexpressionssignatur, die auf ein vermindertes Gesamtniveau der Translation von mRNA hinweist, eines oder mehrere Proteine umfasst, die durch Gene codiert werden, die ausgewählt werden aus der Gruppe, die besteht aus FAM120A, ADAR, MAP1A, COL5A, BCL9L, USP7, RECK, CNOT7, NKIRAS1, ARPC5, CPN31, TMEM208, CRIP2, GDI1, USP9X, ADRM1, PEF1, YIF1B, CANX, GHITM, CANX, TNS1, OGDH, TNPO1 PEPD, HNRNFA3, MAP4, ALDH18A1, DDX21, DERL1, TPM2, NPM1, CLIC4, HLA-A, CDC41, STT3A, RHOA, CTNNB1, TRAM1, PLS3, SSR3, CTSK, NCBP1, PTRH2, SCL35B2, SF3B3, SLC2A14, SCL2A3, NUCB2, HEL-S-109, SEC61G, CLPTM1, RECK, PRMT1, ABCF2, VAMP7, RTCA, AEBP1, CPXM2, S100A16, RRAGD, RRAGC, EHD1 und PALLD.

13. Verfahren nach einem der Ansprüche 9 bis 12, wobei die Versuchsperson ein Träger von G2019S ist oder zu einer Untergruppe von sporadischen PD-Patienten gehört.

14. Verfahren nach einem der Ansprüche 1 bis 13, wobei das Gesamtniveau der Translation von mRNA durch ein Verfahren bewertet wird, das ausgewählt wird aus der Gruppe, die besteht aus Massenspektrometrie, die ausgewählte Reaktionsüberwachung (Selected Reaction Monitoring - SRM), Mehrfachreaktionsüberwachung (Multiple Reaction Monitoring - MRM), parallele Reaktionsüberwachung (Parallel Reaction Monitoring (PRM), datenabhängige Akquisition (Data Dependent Acquisition - DDA) oder datenunabhängige Akquisition (Data Independent Acquisition - DIA) umfasst, metabolischer Markierung und Click-Chemie oder wobei der Wirkstoff ein Antikörper ist.

15. Verfahren nach einem der Ansprüche 1 bis 14, wobei die Proteinexpressionssignatur auf eine nicht vergleichende oder vergleichende Weise verwendet wird.

16. Verfahren nach einem der Ansprüche 1 bis 15, wobei Änderungen bei posttranslationalen Modifikationen, die entweder quantitativ oder qualitativ durch Massenspektrometrie oder Antikörper bewertet werden, auf Änderungen beim Gesamtniveau der Translation von mRNA hinweisen.

17. Verfahren nach Anspruch 16, wobei Änderungen bei der Struktur der posttranslationalen Modifikationen auf ein vermindertes Gesamtniveau der Translation von mRNA hinweisen.

18. Verfahren nach einem der Ansprüche 2 bis 16, wobei das Überwachen das Überwachen des Fortschreitens der PD, das Überwachen der Antwort auf Behandlung, das Überwachen der Remission der PD oder das Überwachen der Wiederkehr der PD umfasst.

19. Verfahren nach einem der Ansprüche 1 bis 17, wobei das Diagnostizieren das Schichten der Versuchsperson in eine Untergruppe der PD umfasst.

20. Verfahren nach einem der vorhergehenden Ansprüche, wobei die Probe eine Hautbiopsieprobe, eine Blutprobe, eine Zellenprobe, eine Gewebeprobe oder eine Körperflüssigkeit ist.

21. Verwendung eines Kits zur Diagnose, Stadienbestimmung oder Überwachung der PD oder zum Schichten von PD-Patienten basierend auf Änderungen bei der Expression von SNX2, FAM120A oder ADAR, wobei das Kit ein oder mehrere Reagenzien zum Testen einer Probe auf Änderungen bei der Expression von SNX2, FAM120A oder ADAR umfasst.

## Revendications

1. Procédé pour diagnostiquer la maladie de Parkinson (MP) chez un sujet, ledit procédé comprenant le test d'un échantillon obtenu auprès du sujet pour des changements du niveau global de traduction d'ARNm en protéines nouvellement synthétisées, dans lequel un niveau global de traduction d'ARNm en protéine nouvellement synthétisée diminué par rapport à celui d'un témoin sain est indicatif d'une MP.

2. Procédé pour surveiller une MP chez un sujet, ledit procédé comprenant le test d'un échantillon obtenu auprès du sujet à différents points de temps pour des changements du niveau global de traduction d'ARNm en protéines nouvellement synthétisées, dans lequel un niveau global de traduction d'ARNm en protéine nouvellement synthétisée diminuant au cours du temps est indicatif d'une progression de la MP.

3. Procédé selon la revendication 1 ou 2, dans lequel une signature d'expression de protéine comprenant une diminution de l'expression de SNX2 est indicative d'un niveau global diminué de traduction d'ARNm.

4. Procédé selon la revendication 3, dans lequel la signature d'expression de protéine indicative d'un niveau global diminué de traduction d'ARNm comprend en outre une diminution de l'expression de LIMA1.

5. Procédé selon la revendication 4, dans lequel la signature d'expression de protéine indicative d'un niveau global diminué de traduction d'ARNm comprend en outre RRS1, TFRC, RASA1, NRAS, KRT36, Q56UQ5, PPIC, TRIM25, JAGN1, WDR36, CSE1L, et RPL27.

6. Procédé selon la revendication 5, dans lequel la signature d'expression de protéine indicative d'un niveau global diminué de traduction d'ARNm comprend en outre MX1, APOL2, DBI, ADA1B0GVG8, ERLEC1, TXNL1, TMSB10, CCDC50, BAG3, RASA1, CHMP4B, EIF2S2, HNRNPH1, NACAGNL3, PSMC1, ACOX1, TMEM167A et ASNS.

7. Procédé selon la revendication 1 ou 2, dans lequel une signature d'expression de protéine indicative d'un niveau global diminué de traduction d'ARNm comprend une ou plusieurs protéines codées par des gènes choisis dans le groupe constitué par SNX2, LIMA1, RRS1, TFRC, RASA1, NRAS, KRT36, Q56UQ5, PPIC, TRIM25, JAGN1, WDR36, CSE1L, RPL27, MX1, APOL2, DBI, ADA1B0GVG8, ERLEC1, TXNL1, TMSB10, CCDC50, BAG3, RA-SA1, CHMP4B, EIF2S2, HNRNPH1, NACAGNL3, PSMC1, ACOX1, TMEM167A et ASNS.

8. Procédé selon l'une quelconque des revendications 1 à 7, dans lequel ladite MP est une MP sporadique.

9. Procédé selon la revendication 1 ou 2, dans lequel une signature d'expression de protéine comprenant une diminution de l'expression de FAM120A et ADAR est indicative d'un niveau global diminué de traduction d'ARNm.

10. Procédé selon la revendication 9, dans lequel la signature d'expression de protéine indicative d'un niveau global diminué de traduction d'ARNm comprend en outre MAP1A, COL5A, BCL9L, USP7, RECK, CNOT7, NKIRAS1, ARPC5, CPN31, TMEM208 et CRIP2.

11. Procédé selon la revendication 10, dans lequel la signature d'expression de protéine indicative d'un niveau global diminué de traduction d'ARNm comprend en outre GDI1, USP9X, ADRM1, PEF1, YIF1B, CANX, GHITM, CANX, TNS1, OGDH, TNPO1 PEPD, HNRNFA3, MAP4, ALDH18A1, DDX21, DERL1, TPM2, NPM1, CLIC4, HLA-A, CDC41, STT3A, RHOA, CTNNB1, TRAM1, PLS3, SSR3, CTSK, NCBP1, PTRH2, SLC35B2, SF3B3, SLC2A14, SLC2A3, NUCB2, HEL-S-109, SEC61G, CLPTM1, RECK, PRMT1, ABCF2, VAMP7, RTCA, AEBP1, CPXM2, S100A16, RRAGD, RRAGC, EHD1 et PALLD.

12. Procédé selon la revendication 1 ou 2, dans lequel une signature d'expression de protéine indicative d'un niveau global diminué de traduction d'ARNm comprend une ou plusieurs protéines codées par des gènes choisis dans le groupe constitué par FAM120A, ADAR, MAP1A, COL5A, BCL9L, USP7, RECK, CNOT7, NKIRAS1, ARPC5, CPN31, TMEM208, CRIP2, GDI1, USP9X, ADRM1, PEF1, YIF1B, CANX, GHITM, CANX, TNS1, OGDH, TNPO1 PEPD, HNRNFA3, MAP4, ALDH18A1, DDX21, DERL1, TPM2, NPM1, CLIC4, HLA-A, CDC41, STT3A, RHOA, CTNNB1, TRAM1, PLS3, SSR3, CTSK, NCBP1, PTRH2, SLC35B2, SF3B3, SLC2A14, SLC2A3, NUCB2, HEL-S-109, SEC61G, CLPTM1, RECK, PRMT1, ABCF2, VAMP7, RTCA, AEBP1, CPXM2, S100A16, RRAGD, RRAGC, EHD1 et PALLD.

13. Procédé selon l'une quelconque des revendications 9 à 12, dans lequel ledit sujet est un porteur de G2019S ou appartient à un sous-groupe de patients souffrant de MP sporadique.

14. Procédé selon l'une quelconque des revendications 1 à 13, dans lequel le niveau global de traduction d'ARNm est déterminé par un procédé choisi dans le groupe constitué par une spectrométrie de masse comprenant une surveillance de réactions sélectionnées (SRM), une surveillance de réactions multiples (MRM), une surveillance de réactions parallèles (PRM), une acquisition dépendante des données (DDA) ou une acquisition indépendante des données (DIA), un marquage métabolique et une chimie clic, ou dans lequel l'agent est un anticorps.

15. Procédé selon l'une quelconque des revendications 1 à 14, dans lequel la signature d'expression de protéine est utilisée d'une manière comparative ou non comparative.

16. Procédé selon l'une quelconque des revendications 1 à 15, dans lequel des changements dans des modifications posttraductionnelles déterminées soit quantitativement soit qualitativement par spectrométrie de masse ou par anticorps sont indicatifs de changements du niveau global de traduction d'ARNm.

17. Procédé selon la revendication 16, dans lequel des changements dans la structure des modifications posttraductionnelles sont indicatives d'un niveau global diminué de traduction d'ARNm.

18. Procédé selon l'une quelconque des revendications 2 à 16, dans lequel ladite surveillance comprend la surveillance de la progression de la MP, la surveillance de la réponse à un traitement, la surveillance d'une rémission de la MP, ou la surveillance d'une rechute de la MP.

19. Procédé selon l'une quelconque des revendications 1 à 17, dans lequel ledit diagnostic comprend la stratification dudit sujet dans un sous-groupe de MP.

20. Procédé selon l'une quelconque des revendications précédentes, dans lequel ledit échantillon est un échantillon de biopsie cutanée, un échantillon de sang, un échantillon de cellules, un échantillon de tissu, ou un fluide corporel.

21. Utilisation d'une trousse pour diagnostiquer, déterminer le stade, ou surveiller une MP, ou stratifier des patients MP sur la base de changements de l'expression de SNX2, FAM120A ou ADAR, dans laquelle ladite trousse comprend un ou plusieurs réactifs pour tester un échantillon pour des changements dans l'expression de SNX2, FAM120A ou ADAR.
